# EUROPEAN PATENT SPECIFICATION

(11) **EP 2 439 540 B1**
(45) Date of publication and mention of the grant of the patent: **21.01.2015**
(21) Application number: 10783528.2
(22) Date of filing: 17.05.2010
(51) Int. Cl.: G01N 35/10, G01N 33/50, A61B 5/15

(54) **SAMPLING/SAMPLE-INJECTING APPARATUS AND BIODATA-MEASURING SET COMPRISING SAME**
PROBENNAHME-/PROBENINJEKTIONSVORRICHTUNG UND BIODATEN-MESSKIT DAMIT
APPAREIL D'ÉCHANTILLONNAGE/D'INJECTION D'ÉCHANTILLON ET ENSEMBLE DE MESURE DE BIODONNÉES COMPRENANT CELUI-CI

(30) Priority: 02.06.2009 KR 20090048804; 15.04.2010 KR 20100034577; 13.05.2010 KR 20100044718
(43) Date of publication of application: 11.04.2012
(73) Proprietor: Infopia Co., Ltd., Gyeonggi-do 431-080 (KR)
(72) Inventor: BAE, Byeong-Woo, Anyang Kyunggi 431-080 (KR); LEE, Sung-Dong, Anyang Kyunggi 431-080 (KR); KHO, Byung-Hoon, Anyang Kyunggi 431-080 (KR); RYU, Ji-Eon, Anyang Kyunggi 431-080 (KR); KIM, Jin-Kyeong, Anyang Kyunggi 431-080 (KR); JOUNG, Hyou-Arm, Anyang Kyunggi 431-080 (KR); AHN, Ku-Cheol, Anyang Kyunggi 431-080 (KR); AHN, Bum-Joo, Anyang Kyunggi 431-080 (KR)
(74) Representative: Gill, Siân Victoria
(86) International application number: PCT/KR2010/003080
(87) International publication number: WO 2010/140779

(56) References cited:
- EP-A1- 0 152 368
- WO-A1-2008/055373
- DE-A1-102006 025 477
- JP-A- 2000 500 671
- JP-A- 2008 157 708
- KR-A- 19990 063 438
- KR-A- 20070 009 732
- US-A- 3 077 780
- US-A- 3 252 331
- US-A1- 2005 269 371

## Description

### Technical Field

The present invention relates generally to a device for collecting a sample and injecting the sample onto a sample receiving area of a measurement strip for measuring biodata, and a biodata-measuring set including the sample collecting device and the measurement strip.

### Background Art

As well known to those skilled in the art, among biometric data measurement devices, there are devices which drop a sample such as blood onto a measurement strip and detect an electrochemical change or a change in color that shows the results of an enzymatic reaction in a reaction zone. Generally, a pipette is used to quantitatively dispense a sample onto the reaction zone of the strip.

However, the pipette is problematic in that its receiving part may suck in an excessive volume of sample, and it is difficult to finely adjust the volume of the received solution that is dispensed. In order to solve these problems, a hole is bored at a predetermined height of a capillary tube part of the pipette for receiving a solution, thus controlling the volume of the received solution. However, the pipette is also problematic in that capillary pressure is higher than the force sucking in the sample at the reaction zone, so that the transfer of the solution to the reaction zone may not be done or may be ceased in the middle of the transfer.

Further, the pipette is frequently made of a flexible material. Since the pipette is apt to bend, the received solution may be undesirably discharged and thus using the pipette is inconvenient.

For these reasons, a sample collecting device capable of adjusting capillary pressure to easily suck in a sample and transfer the received sample to a reaction zone is needed. Also, the sample collecting device has to be able to finely control a volume of the received sample.

Meanwhile, in order to obtain reliable measurement results using a biodata measurement apparatus, it is important to configure a biodata-measuring set (including a sample collecting device and a measurement strip) which is capable of completely filtering out red blood cells from blood so that a sensing part of the biodata measurement apparatus does not show measurement errors due to red blood cells.

Furthermore, in order to rapidly obtain measurement results using the biodata measurement apparatus, the biodata-measuring set is construed to allow a sample to be rapidly transferred to a final reactive layer of the measurement strip.

WO 2008/055373 Al relates to a needle for the discharge of a liquid, as is used in spotting machines, to deposit a liquid onto a carrier plate in the form of points. Said needle consists of a shank which changes into a tapering part. In addition, said needle has a liquid storage in the form of a slot or a reservoir which opens into a grove, in order to guide the liquid to the tip and onto the contact surface.

### Detailed Description of the Invention

### Technical Problem

Accordingly, the present invention has been made keeping in mind the above problems occurring in the prior art, and an object of the present invention is to provide a device for collecting a sample, which is capable of accurately controlling a volume of the received sample, thus making it easy to suck in a sample and to inject the received sample onto a measurement strip, and a biodata-measuring set including the sample collecting device.

Another object of the present invention is to provide a device for collecting a sample, which helps a measurement strip completely filter out red blood cells from a blood sample when the blood sample is injected onto the measurement strip, and a biodata-measuring set including the sample collecting device.

Still another object of the present invention is to provide a device for collecting a sample, which is capable of rapidly measuring biodata by allowing a sample to be rapidly transferred to a final reactive layer of a measurement strip, and a biodata-measuring set including the sample collecting device.

Other additional effects will be described in description of the preferred embodiments.

### Technical Solution

According to a first aspect, there is provided a device for collecting a sample and injecting the sample onto a measurement strip, comprising: a main body; and a sample receiving part protruding from a first end of the main body and having in a top portion a space for receiving the sample by capillary force, wherein an inner wall of the space is treated with a surfactant, wherein the space is formed to diverge in at least two directions from a top central portion of the sample receiving part, when viewed in a front direction, wherein the sample receiving part is formed to diverge in at least two directions from a top central portion of the sample receiving part, when viewed in a front direction, and characterised in that the sample receiving part includes an upper portion formed concavely toward the inside of the sample receiving part, a lower portion formed concavely toward the inside of the sample receiving part while facing the upper portion, and a circumference part connecting the upper portion to the lower portion and formed in a curved shape.

In some embodiments, the sample receiving part is a pair of receiving parts which is open on right, left and bottom sides, and wherein a curvature of the sample receiving part is configured such that an end of the sample receiving part matches a hole formed in a reaction zone of a measurement strip and is in slight contact with the reaction zone.

In some embodiments, in the top central portion of the sample receiving part, a groove is formed having a diameter greater than the spacing distance of the space.

In some embodiments, the top portion of the sample receiving part protrudes in an arc shape or in a globe shape.

In some embodiments, the space is formed in a "-" or "+" when viewed in a front direction.

In some embodiments, in the top central portion of the sample receiving part, a groove whose diameter is greater than a spacing distance of the space is formed.

In some embodiments, the space is graved in a shape whose width is gradually reduced toward the inside of the sample receiving part when viewed in a side direction.

In some embodiments, the space is graved in a shape whose width is gradually reduced toward the inside of the body of the sample receiving part, when viewed in a side direction.

In some embodiments, the groove is formed in a shape whose diameter is gradually reduced toward the inside of the sample receiving device.

In some embodiments, a volume of the space is determined by a volume of the received sample.

In some embodiments, the sample receiving part is made of a transparent material.

According to another aspect, there is provided a biodata-measuring set including: a measurement strip including a sample receiving area onto which a sample is injected; and the sample collecting device of the invention.

### Advantageous Effects

Therefore, when measuring biodata using a measurement strip, the sample collecting device makes it easy to collect and inject a sample, is capable of reducing measurement deviations due to influence of red blood cells, and also leads to rapid measurement of biodata.

### Description of Drawings

FIG. 1 is a schematic view illustrating a biodata measurement apparatus and a measurement strip according to an embodiment of the present invention;
FIG. 2 is a front view illustrating a device for collecting a sample, according to an embodiment of the present invention;
FIG. 3 is a side view illustrating the sample collecting device of FIG. 2;
FIG. 4 illustrates the state wherein a sample receiving part of the sample collecting device of FIG. 2 comes into contact with a reaction zone of a measurement strip, and the flow of the sample received in the sample collecting device;
FIG. 5 is a perspective view of a sample collecting device according to another embodiment of the present invention, and illustrates the state where a receiving piece of the sample collecting device becomes in contact with a reaction zone of a measurement strip when a sample receiving part is formed in a circular shape.
FIG. 6 is a view schematically illustrating a configuration of a biodata-measuring set (including a measurement strip and a sample collecting device) according to an embodiment of the present invention.
FIGS. 7 and 8 are views for explaining operation in which the sample collecting device included in the biodata-measuring set collects a sample.
FIG. 9 is an exploded view illustrating a configuration of the measurement strip included in the biodata-measuring set.
FIG. 10 is a view illustrating an assembled state of the measurement strip illustrated in FIG. 9.
FIG. 11 illustrates paths along which a sample injected onto the measurement strip of FIG. 10 flows.
FIG. 12 illustrates an initial state when a sample is injected onto the measurement strip of FIG. 10.
FIG. 13 illustrates the state of the measurement strip when blood is injected along a normal F1 flow path shown in FIG. 11.
FIG. 14 illustrates the state of the measurement strip when blood is injected along an abnormal F1 flow path shown in FIG. 11.
FIG. 15 is a perspective view illustrating a sample collecting device according to another embodiment of the present invention, included in the biodata-measuring set.
FIGS. 16, 17, and 18 respectively are a side view, a front view, and a plane view illustrating the sample collecting device of FIG. 15.
FIGS. 19 and 20 are views for explaining advantageous effects of the sample collecting device of FIG. 15.
FIG. 21 is a perspective view illustrating a sample collecting device according to another embodiment of the present invention, included in the biodata-measuring set.
FIG. 22 is a view for explaining advantageous effects of the sample collecting device of FIG. 21.
FIG. 23 is a perspective view illustrating a sample collecting device according to another embodiment of the present invention, included in the biodata-measuring set.
FIG. 24 is a view for explaining advantageous effects of the sample collecting device of FIG. 23.
FIG. 25 is a perspective view illustrating a sample collecting device according to another embodiment of the present invention, included in the biodata-measuring set.
FIG. 26 is a view for explaining advantageous effects of the sample collecting device of FIG. 25.
FIG. 27 is a perspective view illustrating a sample collecting device according to another embodiment of the present invention, included in the biodata-measuring set.
FIGS. 28 through 37 are perspective views illustrating various modified embodiments.

### Best Mode

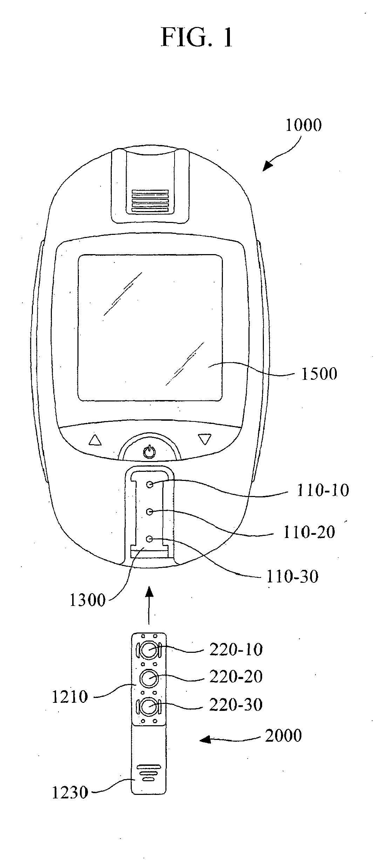

FIG. 1 is a schematic view illustrating a biodata measurement apparatus 1000 and a measurement strip 2000 according to an embodiment of the present invention. Referring to FIG. 1, the biometric data measurement device 1000 includes a power button, a strip insertion part 1300, and a display part 1500. A groove is formed inside the edge of the strip insertion part 1300, thus allowing the measurement strip 2000 to be easily inserted into and secured to the strip insertion part 1300.

Further, the strip insertion part 1300 includes a plurality of sensing parts 110-10, 110-20 and 110-30 which are provided on the central portion of the strip insertion part 1300 in such a way that they are spaced apart from each other. The sensing parts 110-10, 110-20 and 110-30 form a one-to-one correspondence with reaction zones of the measurement strip 2000 or with sample receiving holes 220-10, 220-20, and 220-30. All or some of the sensing parts 110-10, 110-20 and 110-30 are activated according to a measured type, thus sensing the reaction zones of the measurement strip 2000.

The measurement strip 2000 includes a plurality of reaction zones for measuring biometric data, such as the volume of neutral fat or cholesterol which is present in blood. According to the position of each reaction zone, different data is measured. The measurement strip 2000 has on its upper portion a protrusion which is inserted into the groove of the strip insertion part 1300, thus enabling the measurement strip 20 to be easily secured to the strip insertion part 1300.

Further, the measurement strip 2000 includes an upper support 1210 and a lower support 1230 which are coupled to each other. The reaction zones are provided between the upper support 1210 and the lower support 1230, and comprise paper to which a catalyst is coupled. The upper support 1210 includes holes 220-10, 220-20 and 220-30 through which a portion of a reaction part, namely, the reaction zones, are exposed, so that a sample such as bodily fluid can be applied to the reaction zones and produce a biological response. Here, the holes of the upper support 1210 are formed to make predetermined angles with respect to the reaction zones.

In order to perform reaction in the reaction zones of the measurement strip 2000, a reaction solution such as blood must be accurately injected into the reaction zones.

Conventionally, bodily fluid such as collected blood has been injected using a pipette. In the case of using a pipette, an excessive volume may be injected into the reaction zones and it is difficult to control a dispensing volume. Further, since the capillary pressure of the pipette is higher than the pressure of the reaction zones, the solution may not be transferred into the reaction zones, or the transfer of the solution may be ceased. A device for collecting a sample according to the present invention is proposed to solve these problems.

Hereinafter, a device for collecting a sample according to an embodiment of the present invention will be described in detail.

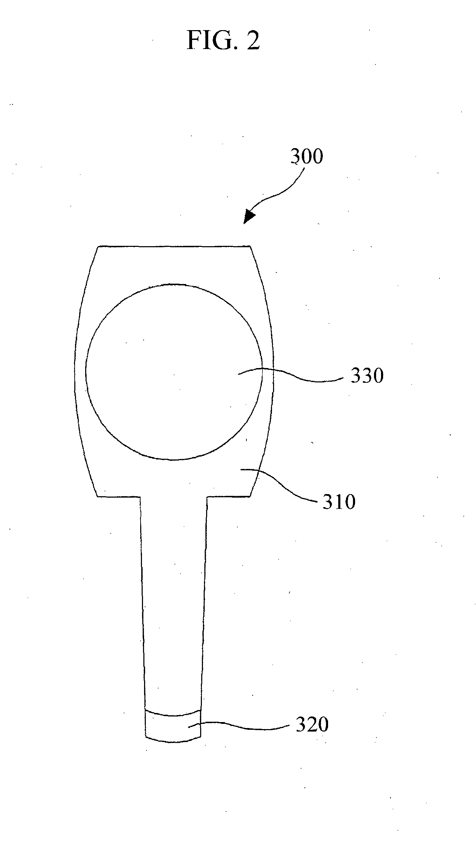

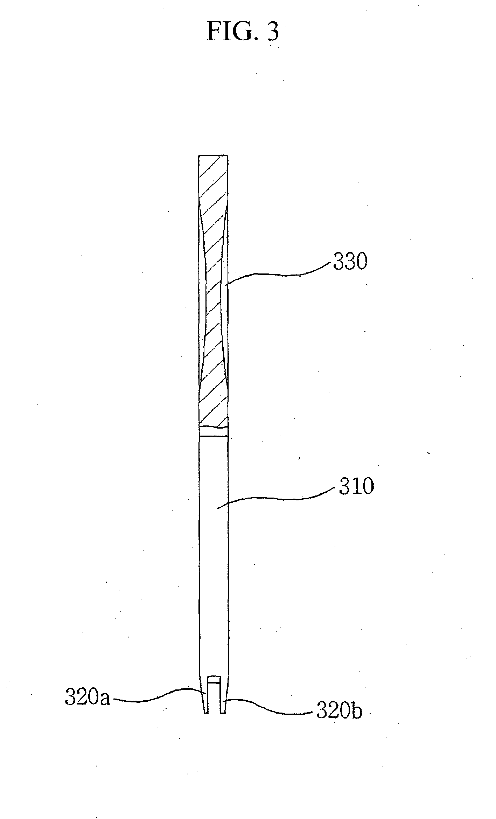

FIG. 2 is a front view illustrating a device for collecting a sample according to an embodiment of the present invention, and FIG. 3 is a side view illustrating the sample collecting device of FIG. 2.

As shown in the drawings, the sample collecting device 300 according to this embodiment includes a main body 310 and a receiving piece 320.

The main body 310 may be made of an acrylic material. According to this embodiment, the main body 310 includes one pair of finger seats 330 which are formed to be concave to allow a user to easily hold the device 300 with his or her finger. The finger seats 330 allow a user to easily hold the device with his or her thumb and forefinger, thus preventing the sample collecting device from slipping or falling out of his or her hand.

The sample receiving part 320 are integrated with the main body 310, and protrude from one end of the main body 310 in such a way as to be parallel to each other and be spaced apart from each other, with a space defined between the sample receiving part 320 to receive a solution. According to this embodiment, the right and left and bottom of the receiving piece 320 are open, thus defining a space which receives the sample by capillary force acting on the space between the sample receiving part 320. For example, the receiving piece 320 may be made of an acrylic or plastic material, as is the main body 310.

Further, as shown in FIG. 3, the sample receiving part 320a and 320b are provided to be parallel from and spaced apart from each other, and the right and left and bottom of the space between the sample receiving part are open, thus allowing the sample such as blood to easily flow into a receiving area.

According to this invention, the sample receiving part 320a and 320b may not be parallel to each other but may be formed such that a spacing distance between the sample receiving part 320a and 320b is reduced or increased in a direction distant from the main body 330. Preferably, the spacing distance between the sample receiving part 320a and 320b ranges from 0.20mm to 2.0mm.

Further, the receiving area is determined according to the height of the receiving piece 320. Thus, it is possible to control the volume of the received sample by adjusting a width of the receiving piece 320 which protrudes from the main body 310. In this embodiment, the receiving piece 320 is constructed to form the receiving area which may receive a sample of 10µℓ or less. That is, the width of the receiving piece 320 is designed to match the volume of the received solution, thus preventing capillary force from being generated because of air being in the space where there is no solution. Thus, the solution received in the receiving area may be easily transferred to the reaction zones of the strip.

Here, the pair of sample receiving part 320 may differ in height. Although the sample receiving part 320a and 320b have different heights, it is preferable that the difference in height be 3mm or less.

Further, according to an aspect of the present invention, the first end of the main body 310 protrudes in the shape of an arc, and one pair of sample receiving part 320 protrudes in the shape of an arc to correspond to the shape of the first end of the main body 310.

Preferably, the receiving piece 320 is formed to be convex in the direction of outflow of the sample, thus allowing the sample, such as bodily fluid, received in a confined space to be easily transferred to the measurement strip.

The curvature of the receiving piece 320 is set such that an end of the receiving piece 320 matches the hole formed in the reaction zone of the measurement strip and makes slight contact with the reaction zone. Here, the receiving piece 320 is inclined to correspond to the shape of the hole of the measurement strip through which the reaction zone is exposed, so that at least a portion of the receiving piece 320 is in contact with the reaction zone of the measurement strip.

Further, according to another aspect of this invention, the inner wall of the receiving piece 320 is treated with a surfactant. Preferably, the inner wall of the receiving piece 320 is treated with a non-ionic surfactant so that red blood cells are not broken. Thereby, the sample such as blood may be more rapidly absorbed between the sample receiving part.

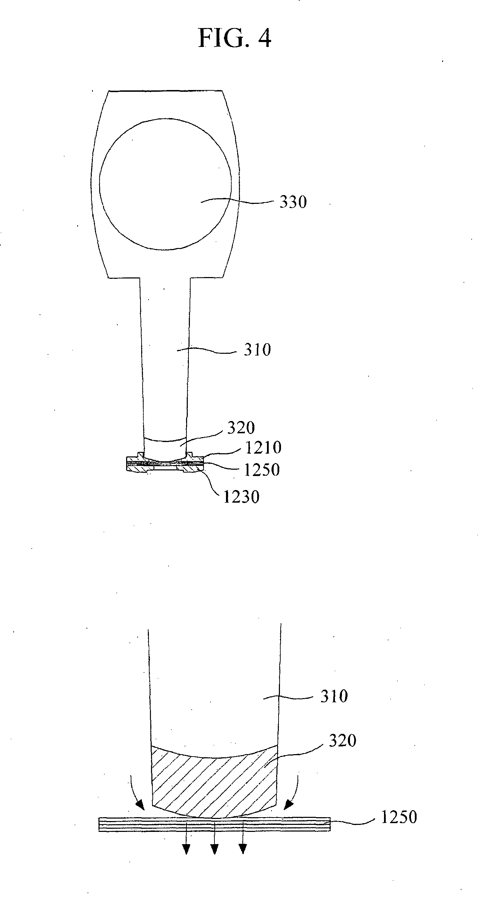

The upper part of FIG. 4 illustrates the state wherein the receiving piece of the sample collecting device of FIG. 2 comes into contact with the reaction zone of the strip.

As shown in FIG. 4, in the sample collecting device according to this embodiment, the receiving piece 320 has a circular shape to match the shape of the hole formed in the upper support 1210 of the measurement strip, so that the central portion of the receiving piece 1320 is in contact with a reaction part 1250 which is inserted between the upper support 1210 and the lower support 1230. Thus, the sample collecting device enables the reaction part 1250 to accurately absorb the received solution.

Since the upper layer of the reaction zone of the strip has good sample dispersion and is dry, the capillary force of the reaction zone of the strip is larger than that of the internal receiving space of the receiving piece 320 of the sample collecting device. Therefore, when the central portion of the receiving piece 320 of the sample collecting device comes into contact with the reaction zone of the strip, the sample between the sample receiving part 320 can be rapidly transferred to the reaction zone of the strip.

The lower part of FIG. 4 illustrates the flow of the sample received in the sample collecting device. As shown in the drawing, the sample receiving part 320 protrude to be parallel to and spaced apart from each other, and the bottom and both sides of the receiving space defined by the sample receiving part 320 are open, thus allowing a sample such as blood to more easily suck in the receiving space. Further, an end of the sample receiving part 320 protrudes in the shape of an arc, thus making it easy for the received solution to flow in the direction shown by the arrows. Since the height of the receiving space defined by the sample receiving part 320 is not high, an excessive increase in capillary force is prevented. Thus, the collected sample of the receiving space can be more smoothly absorbed by the reaction part 250.

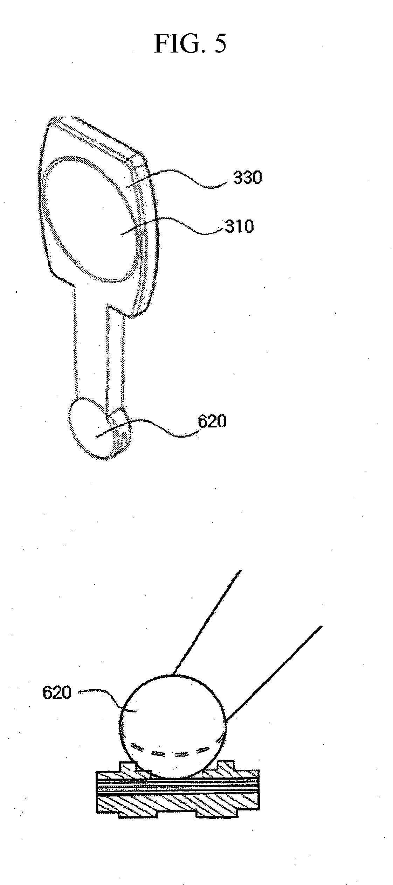

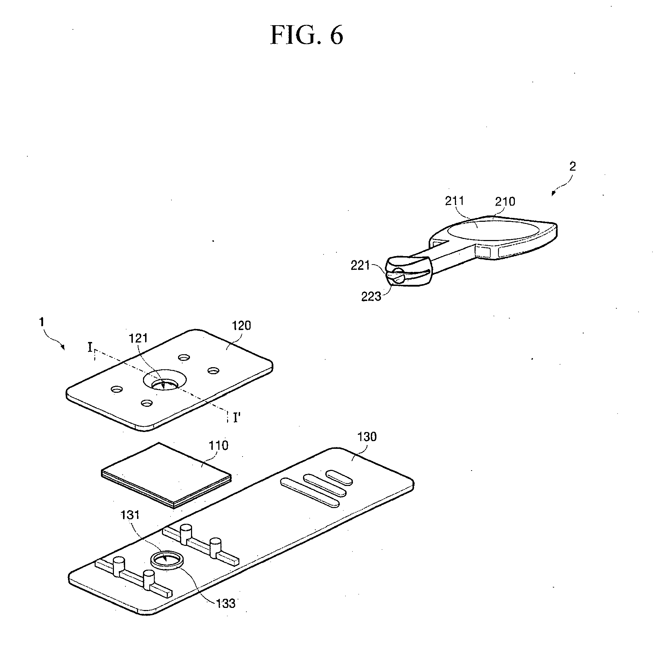

The upper part of FIG. 5 is a perspective view illustrating a device for collecting a sample, according to another embodiment of the present invention, and the lower part of FIG. 5 illustrates the state wherein a receiving piece of the sample collecting device of FIG. 6 comes into contact with a reaction zone of a strip.

Referring to the drawings, the sample collecting device according to another embodiment of the present invention includes a circular receiving piece 620.

As shown in the drawing, the receiving piece 620 has a circular shape, and the width of the receiving piece 620 is variably adjustable, so that the receiving piece 620 may receive a desired sample to the extent of about 20µℓ. In the manufacturing process, the width of the receiving piece 620 may be quantitatively determined according to the volume of the received sample.

Further, the receiving piece 620 has the circular shape described above. Thus, as shown in FIG. 5, although the receiving piece 620 obliquely comes into contact with the reaction zone, it is possible to inject the received sample. Since the receiving piece 620 doesn't have to come into contact with the reaction zone in a vertical direction, convenience is improved. According to an embodiment, although the sample collecting device comes into contact with the reaction zone to be inclined to the right or left by 60 degrees with respect to a vertical angle, the received sample can be injected into the reaction zone.

As described above, the present invention provides a device for collecting a sample, which enables the injection of samples such as blood. Further, the sample collecting device allows the received sample to be easily transferred to a reaction zone of a measurement strip, and enables the sample to be completely absorbed by the reaction zone because of capillary force in a receiving area when the device comes into contact with the reaction zone of the strip.

Further, according to the depth of the space for receiving the solution, the volume of the received solution may be variously adjusted, so that it is possible to inject and receive an accurate volume of solution.

Moreover, a sample can be more smoothly absorbed by a receiving area, and the transfer of the received sample to a reaction zone is easy although a sample collecting device receiving the sample is in contact with the reaction zone at various angles.

Hereinafter, sample collecting devices according to other improved embodiments of the present invention will be described. In the following descriptions, the advantages of the sample collecting devices will be described together with measurement strips that are respectively used with the corresponding sample collecting devices.

First, FIG. 6 is a view schematically illustrating a configuration of a biodata-measuring set (including a measurement strip 1 and a sample collecting device 2) according to an embodiment of the present invention. As shown in FIG. 6, the biodata-measuring set includes the measurement strip 1 configured to cause a change in color or an electrochemical change when a sample such as blood is injected thereon, and the sample collecting device 2 for collecting a sample (for example, blood) from a user and injecting the blood sample onto the measurement strip 1. According to a preferred embodiment, the measurement strip 1 may be construed to be inserted into and secured to a separate biodata-measuring apparatus (not shown) which analyzes the results of measurements by the measurement strip 1.

The measurement strip 1 includes a measurement layer 110 configured to cause a change in color or an electrochemical change when reacting with a sample, a upper cover 120 having a an opening 121 to expose a portion of the upper surface of the measurement layer 110, and a lower substrate 130 having an opening 131 to expose a portion of the lower surface of the measurement layer 110.

The sample collecting device 2 includes a main body 210 having a pair of finger seats 211 formed to allow a user to easily hold the sample collecting device 2, and a sample receiving part 220 extended from the main body 210 and having on a top portion a space 221 through which a sample is injected by capillary force. Meanwhile, in the central portion of the space 221, a groove 223 is formed to make direct contact with the upper surface of the measurement layer 110 of the measurement strip 1.

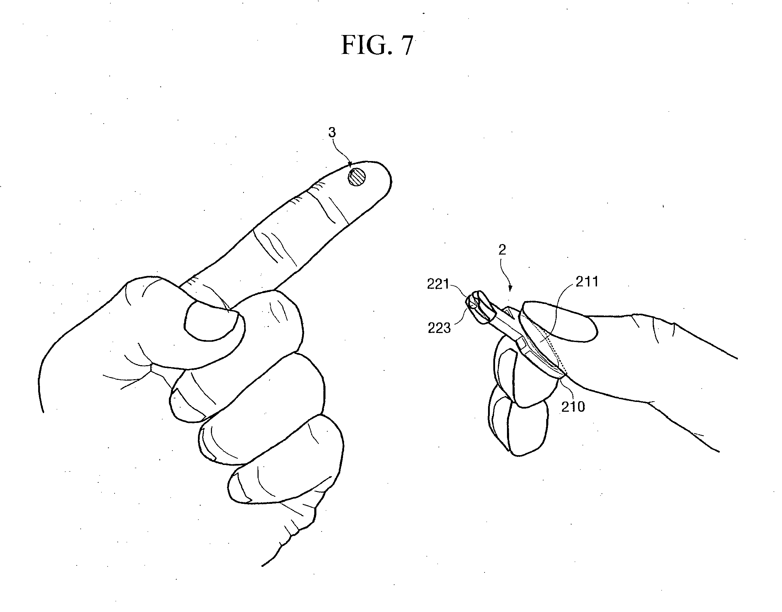

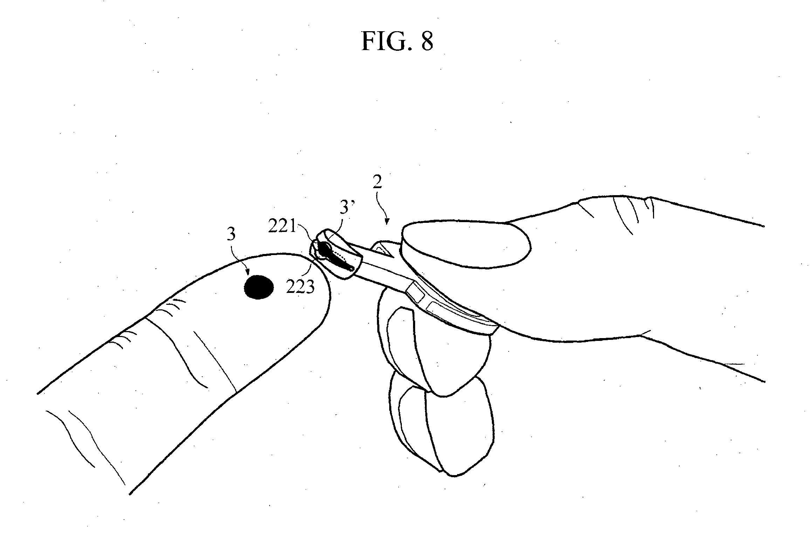

Now, the sample collecting device 2 will be described in more detail with reference to FIGS. 7 and 8. As shown in FIGS. 7 and 8, when a user brings the space 221 and groove 223 into contact with a sample (for example, blood 3) while holding the seats 211 with his or her thumb and forefinger, the sample collecting device 2 sucks in the blood 3 toward the main body 210 by capillary force. Thereafter, the sample collecting device 2 easily and rapidly injects blood 3' received in the space 221 onto the measurement layer 110 (see FIG. 6) because of the relatively wide width of the space 221, which will be described later.

According to a preferred embodiment, the sample collecting device 2 may be made of one material of transparent Polymethyl Methacrylate (PMMA), acrylics, Polycarbonate, transparent plastic. A user can check with his or her eye the state where the sample (for example, blood) is injected into the sample collecting device 2 or where the sample is injected from the sample collecting device 2 to the measurement layer 110.

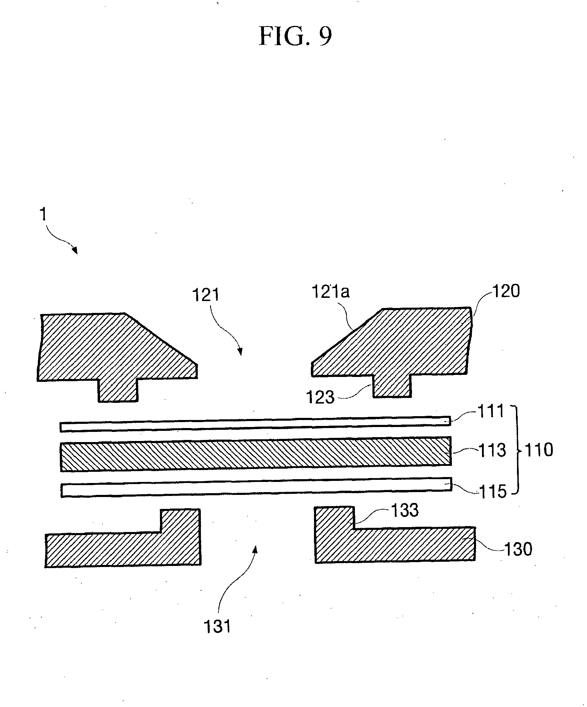

Hereinafter, a measurement strip 1 that can be applied to or included in the biodata-measuring set according to the present invention will be described in detail with reference to FIGS. 9 through 14.

FIG. 9 is an exploded view illustrating a configuration of the measurement strip 1 that can be applied to or included in the biodata-measuring set. FIG. 9 corresponds to a cross-sectional view cut along a line I-I' of FIG. 6.

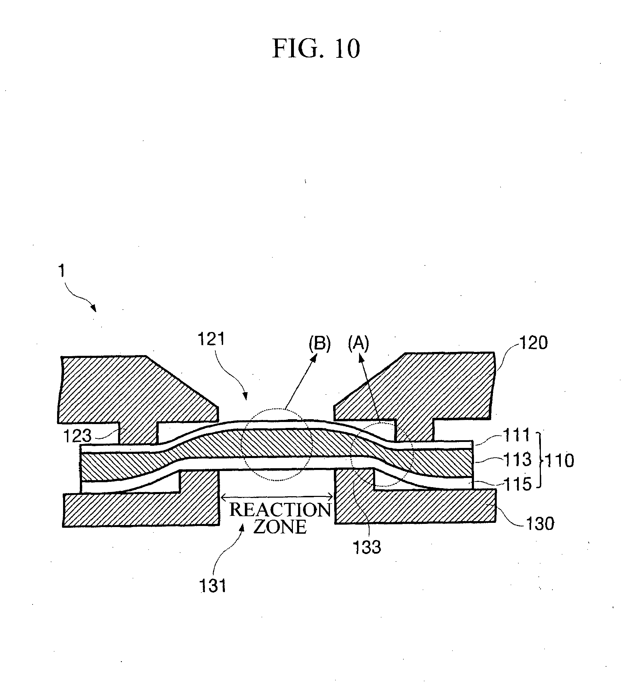

FIG. 10 is a view illustrating an assembled state of the measurement strip 1 illustrated in FIG. 9. First, referring to FIG. 9, the measurement strip 1 includes a measurement layer 110, a upper cover 120, and a lower substrate 130. The measurement layer 110 is used to determine reaction with a sample, and causes a change in color or an electrochemical change when reacting with a sample such as blood. The measurement layer 110 may be implemented by stacking a diffusive layer 111, a filtering layer 113, and a reactive layer 115 in this order. The diffusive layer 111 allows a sample, such as blood or plasma, to rapidly and uniformly be diffused. The diffusive layer 111 may be made of a woven material, such as polyester and cotton, or a non-woven fabric, such as fabric, gauze and monofilament.

The filtering layer 113 is disposed below the diffusive layer 111, and functions as a filter for filtering out blood cells such as red blood cells from the sample (for example, blood) spreading by the diffusive layer 111. For example, the filtering layer 113 filters out blood cells from the blood inflowing from the diffusive layer 111, and then transfers only serum of the blood from which blood cells have been removed to the reactive layer 115. According to a preferred embodiment, the filtering layer 113 may be implemented in the form of a pad containing glass fibers. However, the pad may be made of polyester, nitrocellulose, or poly-sulfonate.

The reactive layer 115 contains dry chemicals and reactance, and causes a change in color when reacting with cholesterol or the like.

The upper cover 120 has an opening 121 whose edge 121 is inclined with a constant curvature, and the lower side of the upper cover 120 has a protrusion 123 protruding toward the lower substrate 130. The protrusion 123 presses the measurement layer 110 downward after assembling.

The lower substrate 130 has an opening 131 in correspondence to the opening 121 of the upper cover 120, and the edge portion of the opening 131 forms a protrusion 133 protruding in the direction of the upper cover 120. The protrusion 122 presses the measurement layer 110 upward after assembling.

Then, referring to FIG. 10, since the upper protrusion 123 and the lower protrusion 133 press the measurement layer 110 downward and upward while being interlaced with each other, the measurement layer 110 is formed to have a convex upper portion and a flat lower portion.

Also, since the downward and upward pressing forces by the protrusions 123 and 133 are directly transferred to an area A where the upper protrusion 123 is interlaced with the lower protrusion 133, the diffusive layer 111, the filtering layer 113, and the reactive layer 115 configuring the measurement layer 110 make close contact with each other. On the contrary, in an area B corresponding to the central portion of the opening 121, the downward and upward pressing forces by the protrusions 123 and 133 are weakened, and accordingly the degree of close contact between the diffusive layer 111, the filtering layer 113, and the reactive layer 115 configuring the measurement layer 110 is relatively low.

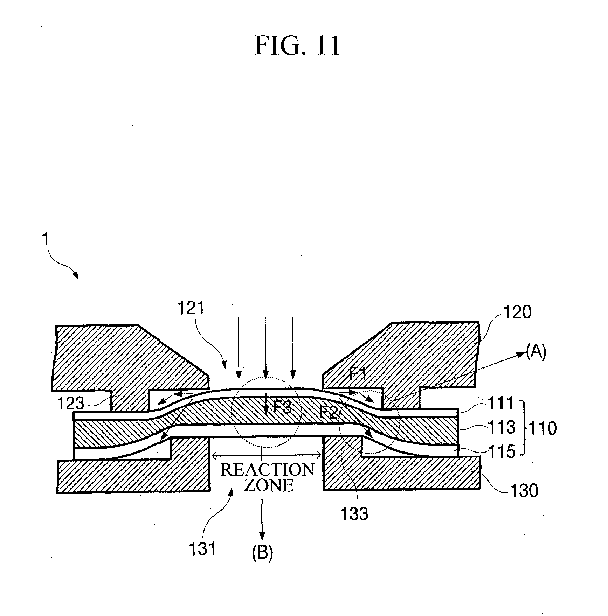

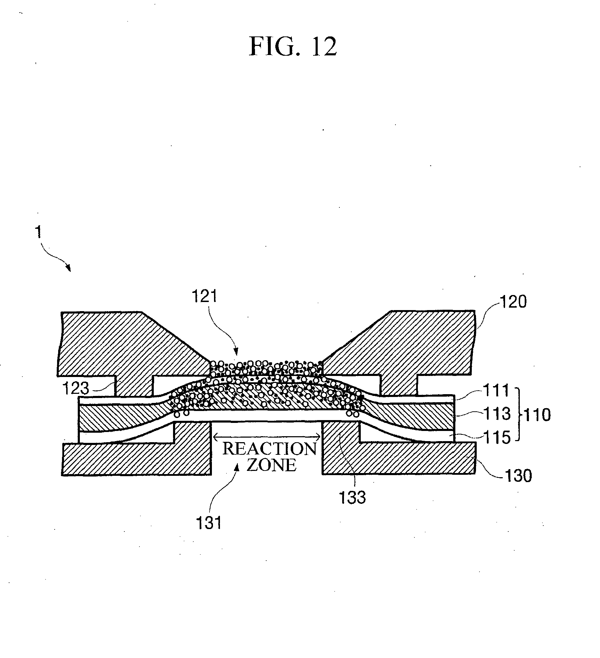

FIG. 11 illustrates paths along which a sample injected into the measurement strip 1 flows, and FIG. 12 illustrates an initial state when a sample is injected into the measurement strip 1.

As shown in FIG. 11, in the outside of the opening 121 of the measurement strip 1 applied to or included in the biodata-measuring set according to the present invention, a flow F1 of a sample that is absorbed in the lower portion of the measurement layer 110 and a flow F2 of the sample flowing out of the measurement area are generated simultaneously. However, since the A area (see FIG. 10) where the upper and lower protrusions 123 and 133 are interlaced with each other is subject to a strong pressing force by the upper and lower protrusions 123 and 133, the FI flow is a main flow, and the F2 flow is a relatively small flow. Also, in the B area (see FIG. 10) corresponding to the central portion of the opening 121, a flow F3 of the sample that is absorbed in the lower portion of the measurement layer 110 is very limited although the flow exists.

As shown in FIG. 12, just when blood is injected through the opening 121 of the measurement strip 1, a relatively large amount of blood is loaded to the outside of the opening 121 due to the convex shape of the measurement layer 110. Also, most of the blood loaded to the outside of the opening 121 spreads (by the diffusive layer 111) and is subject to removal of blood cells (by the filtering layer 113) along the path of the F1 flow, and then finally reaches the reactive layer 115 (a vertical flow).

Thereafter, the serum of the blood from which blood cells have been removed spreads in the direction of the inside or outside portion of the reaction zone (a horizontal flow). At this time, since the area (the A area of FIG. 11) where the upper and lower protrusions 123 and 133 are interlaced with each other is subject to a strong pressing force by the protrusions 123 and 133, most of the serum inflows to the inside portion of the reaction zone and the amount of the serum flowing out to the outside portion of the reaction zone is very small.

Meanwhile, when the serum continues to inflow to the reactive layer 115 (a vertical flow) and the inflowing serum continues to be transferred to the inside portion of the reaction zone along the F1 flow path so that a portion corresponding to the reaction zone in the reactive layer 115 reaches a saturated state, the serum flow (a vertical flow) to the inside portion of the reaction zone is ceased.

Also, when the serum flow to the inside portion of the reaction zone in the reactive layer 115 is ceased, the excessive serum flows out of the reaction zone, so that the amount of the sample accumulated in the portion corresponding to the reaction zone in the reactive layer 115 is maintained constant.

Meanwhile, the area (the B area of FIG. 11) corresponding to the central portion of the opening 121 in the measurement layer 110 loads a relatively smaller amount of blood than the outside area does (because of the convex shape of the upper portion of the measurement layer 110). Also, as described above with reference to FIG. 10, since in and around the area (the B area of FIG. 11) corresponding to the central portion of the opening 121, a degree of close contact between the diffusive layer 111, the filtering layer 113, and the reactive layer 115 is relatively lower than that of the outside area (the A area of FIG 11), a degree at which blood flowing along the path of the F3 flow contributes to serum saturation is very small.

As such, in the measurement strip 1 applied to or included in the biodata-measuring set, blood flowing along the F1 flow path directly contributes to serum saturation of the reactive layer 115.

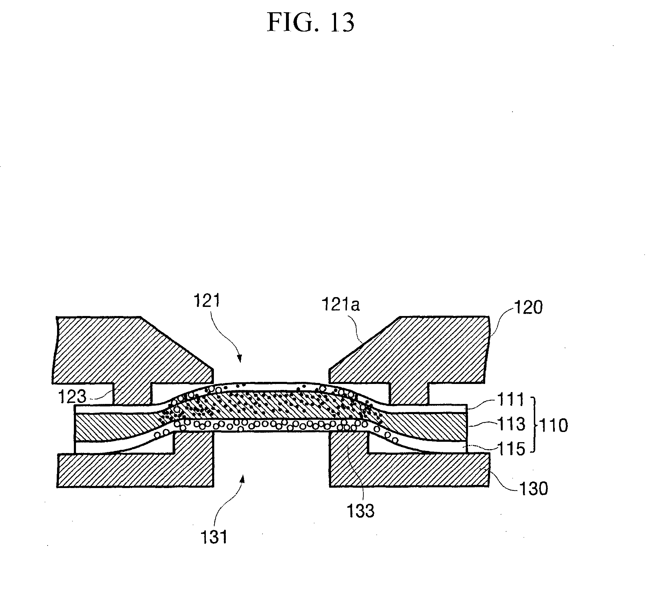

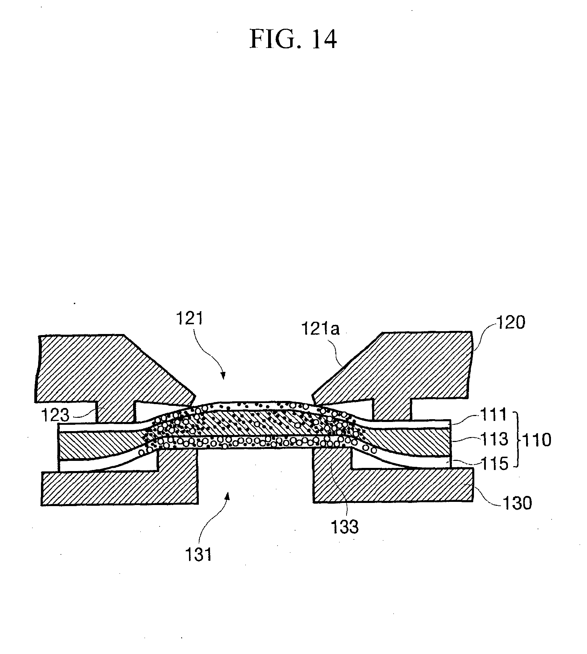

FIG. 13 illustrates the state of the measurement strip 1 when blood is injected along a normal F1 flow path (see FIG. 11), and FIG. 14 illustrates the state of the measurement strip 1 when blood is injected along an abnormal F1 flow path.

As shown in FIG. 13, in the measurement strip 1, when blood is injected along a normal path, the injected blood is diffused by the diffusive layer 111 and the blood cells of the diffused blood are filtered out, so that only the serum of the blood is transferred to the reactive layer 115.

However, there are cases where the F1 flow path changes, for example, due to pressure, etc. applied locally to the edge portion 121a of the opening 121 upon injection of blood to the measurement strip 121. For example, when the edge portion 121a of the opening 121 is locally pressed downward and the measurement layer 110 below the edge portion 121 is compressed, blood cells (for example, red blood cells) remaining in the filtering layer 113 may forcibly enter the reactive layer 115.

FIG. 14 shows the case where a part of the edge portion 121a is strongly pressed downward by a sample collecting device, etc., and thus the FI flow path changes. That is, when a part of the edge portion 121a is strongly pressed by a sample collecting device, etc. while blood is injected, a phenomenon where red blood cells, etc. having to be filtered out by the filtering layer 113 enter the reactive layer 113 by the pressure occurs. The red blood cells that forcibly enter the reactive layer 115 act as a main factor that makes wrong measurement results.

Hereinafter, a sample collecting device capable of suppressing red blood cells from being transferred to a reactive layer will be described. Also, a configuration of a sample collecting device capable of more rapidly injecting blood will be described.

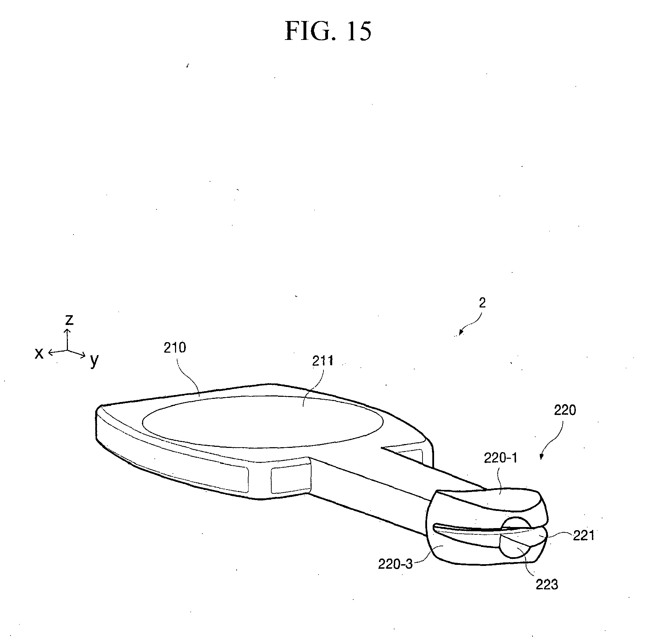

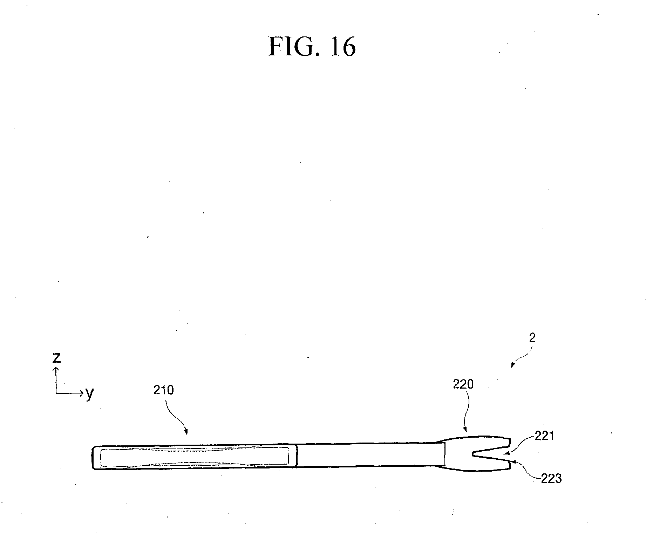

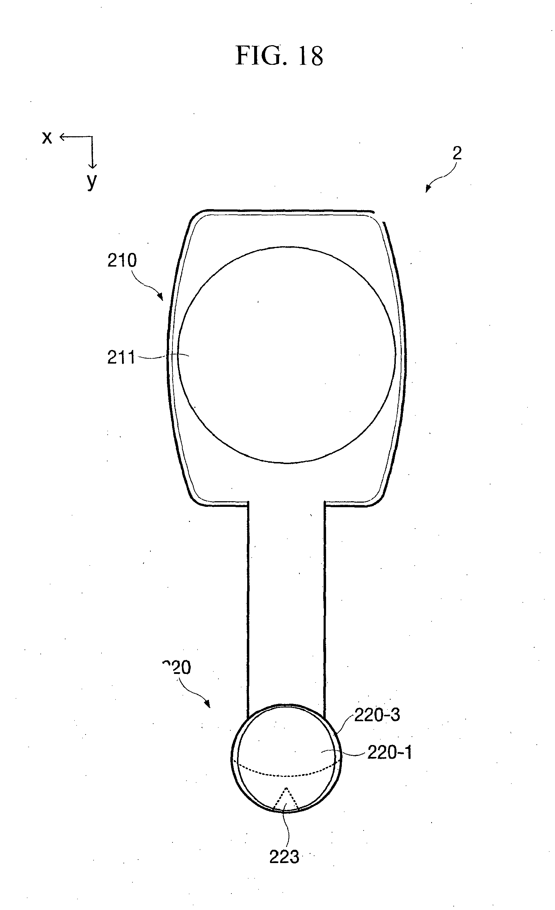

FIG. 15 is a perspective view illustrating a sample collecting device 2 according to another embodiment of the present invention, applied to or included in the biodata-measuring set, and FIGS. 16, 17, and 18 respectively are a side view, a front view, and a plane view illustrating the sample collecting device 2 of FIG. 15. In the following descriptions, the x-axis direction denoted in FIG. 15 is defined as a side direction of the sample collecting device 2, the y-axis direction is defined as the front direction of the sample collecting device 2, and the z-axis direction is defined as the plane direction of the sample collecting device 2. The sample collecting device 2 according to another embodiment of the present invention has the same structure as the sample collecting device 300 illustrated in Fig. 2, except for the structure of a sample receiving part 220.

The sample collecting device 2 includes a main body 210 having a pair of finger seats 211 formed to be concave to allow a user to easily hold the sample collecting device 2, and a sample receiving part 220 extending from the main body 210 and having at the top portion a space through which a sample 221 (for example, blood) is received by capillary force.

In the sample receiving part 220, a upper side 220-1 and a lower side 220-2 opposite to the upper side 220-1 are formed to be concave, and a circumference part 220-3 coupling the upper side 220-1 with the lower side 220-2 is curved with a predetermined curvature.

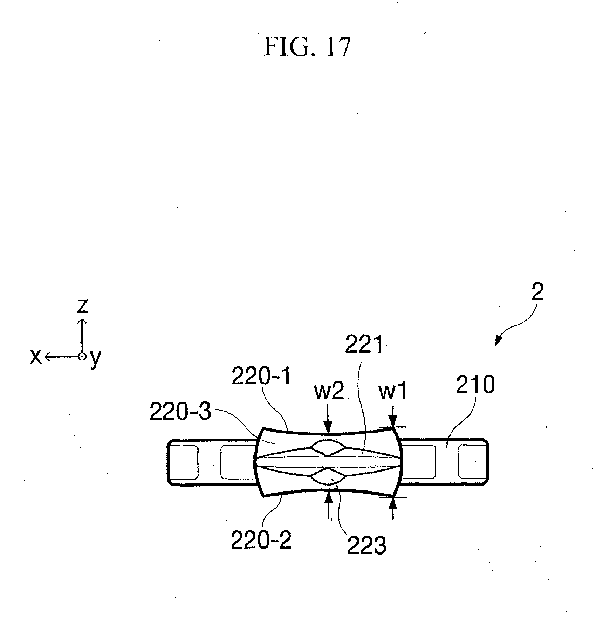

The width W1 of each end portion of the circumference part 220-3 is, when viewed in the front directed, greater than the width W2 of the central portion of the circumference part 220-3 (see FIG. 17).

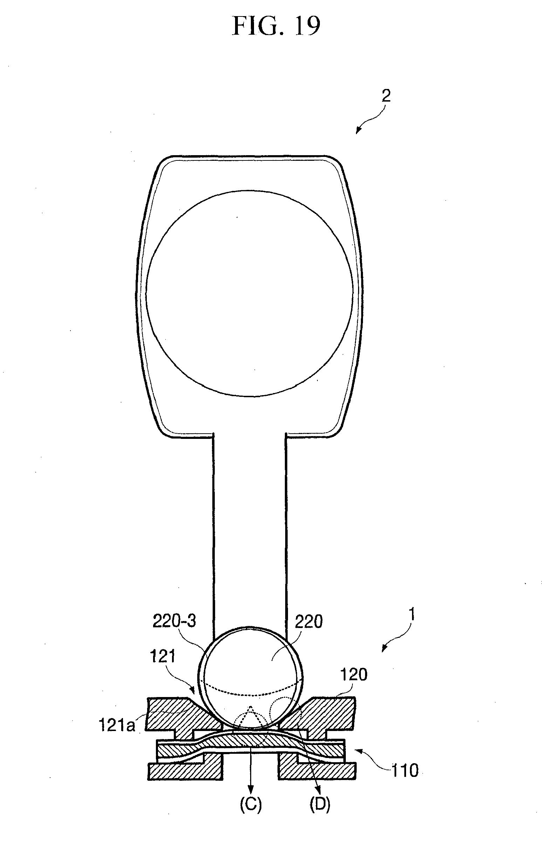

Also, in this embodiment, the curvature of the circumference part 220-3 matches the curvature of the edge portion of the opening 121. Accordingly, when injecting blood onto the measurement layer 110 of the measurement strip 1 through the sample collecting device 2, a user can securely rest the circumference part 220-3 of the sample receiving part 220 on the edge portion 121 of the opening 121 (see FIG. 19).

The space 121 formed in the sample receiving part 220 is, when viewed in the front direction, graved to become in a "-" shape from the left to the right. Specifically, the space 221 of the sample receiving part 220 is, when viewed in the side direction, formed in a "V" shape, the width of the space 221 being gradually narrowed toward the inside. Accordingly, when blood is collected through the sample collecting device 2, the blood can be completely suck into the inside of the sample receiving part 220 without remaining any blood stains on the circumference part 220-3. Meanwhile, the "V" shape is further advantageous because it can be easily molded upon injection molding.

Also, in the top central portion of the sample receiving part 220, a groove 223 whose diameter is greater than the spacing distance of the space 331 is formed. The groove 223 makes direct contact with the upper surface of the measurement layer 110 of the measurement strip 1. Accordingly, when blood is injected through the sample collecting device 2, blood received in the space 221 of the sample receiving part 220 can be rapidly transferred to the measurement layer 110 of the measurement strip 1 through the groove 223 having a relatively wide width. According to a preferred embodiment, the groove 223 may be formed in a cone shape whose diameter is gradually reduced from the top portion to the end portion.

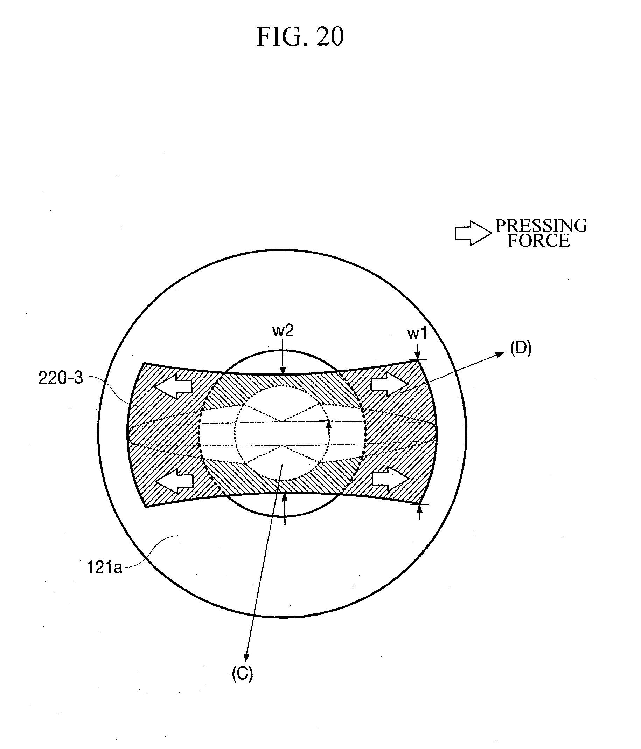

FIGS. 19 and 20 are views for explaining advantageous effects of the sample collecting device of FIG. 15.

First, referring to FIG. 19, the sample collecting device 2 has a groove 223 whose diameter is greater than the spacing distance of the space 221, and the groove 23 also becomes in direct contact with the upper surface of the measurement layer 110 of the measurement strip 1, so that blood received in the sample receiving part 220 can be more smoothly and rapidly transferred to the measurement strip 1 (that is, to the measurement layer 110). Accordingly, for example, a biodata-measuring apparatus can more rapidly acquire the measurement results by the measurement strip 1.

Also, in the sample collecting device 2 according to the present embodiment, since the curvature of the circumference part 220-3 matches the curvature of the edge portion 121a of the opening 121, it is possible to securely rest the circumference part 220-3 of the sample receiving part 220 on the edge portion 121a of the opening 121. For example, although a user brings the sample collecting device 2 into contact with the opening 121 of the measurement strip 1 at various angles, he or she can securely rest the sample receiving part 220 on the edge portion 121a of the opening 121.

Then, FIG. 20 is a view for explaining the state where the sample receiving part 220 (specifically, the circumference part 220-3) of the sample collecting device 2 is rested on the edge portion 121a of the opening 121. An area C of FIG. 20 is an area where the groove 223 of the sample collecting device 2 is in contact with the measurement layer 110 of the measurement strip 1, and an area D is an area where the circumference part 220-3 of the sample collecting device 1 is in contact with the edge portion 121a of the measurement strip 1.

Referring to FIG. 20, in the sample collecting device 2 according to the present embodiment, since the width W1 of each end portion of the circumference part 220-3 is, when viewed in the front direction, greater than the width W2 of the central portion of the circumference part 220-3, a pressing force transferred from the circumference part 220-3 of the sample collecting device 2 to the edge portion 121a is distributed uniformly over the entire area of the edge portion 121a. As described above with reference to FIG. 14, when a pressure is applied to a part of the edge portion 121a of the opening 121, the pressed part of the edge portion 121a is compressed downward, and accordingly the F1 flow path directly contributing to serum saturation of the reactive layer 115 of the measurement strip 1 may change. However, in the case of using the sample collecting device 1 according to the present embodiment, since a pressing force transferred from the circumference portion 220-3 of the sample collecting device 1 to the edge portion 121a is distributed uniformly over the entire area of the edge portion 121a of the opening 121, the phenomenon where the edge portion 121a is locally pressed downward can be effectively prevented.

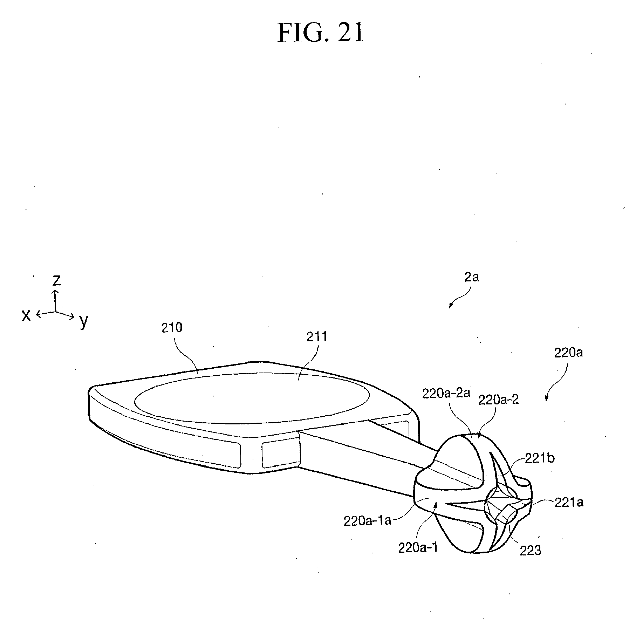

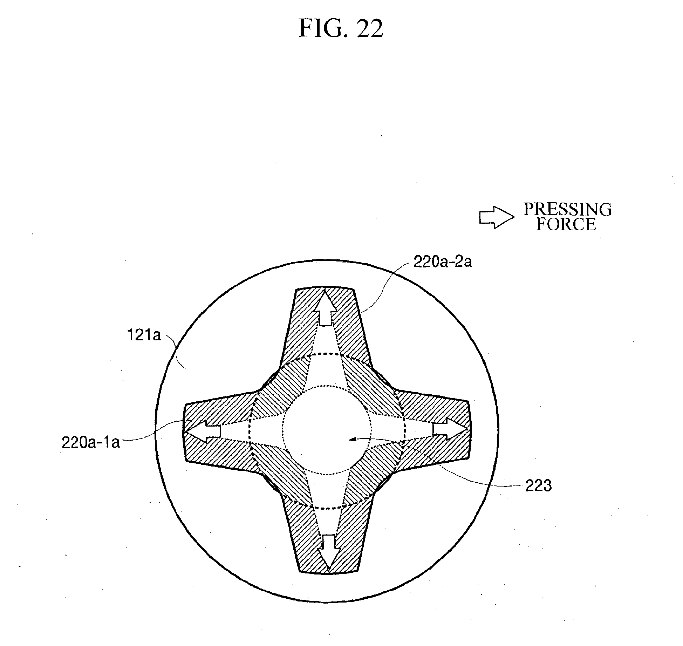

FIG. 21 is a perspective view illustrating a sample collecting device 2a according to another embodiment of the present invention, applied to or included in the biodata-measuring set, and FIG. 22 is a view for explaining advantageous effects of the sample collecting device of FIG. 21.

The sample collecting device 2a according to this embodiment has the same structure as the sample collecting device 2 illustrated in FIG. 15, except for the structure of a sample receiving part 220a.

Meanwhile, in FIG. 21, the x-axis direction is defined as the side direction of the sample collecting device 2a, the y-axis direction is defined as the front direction of the sample collecting device 2a, and the z-axis direction is defined as the plane direction of the sample collecting device 2a.

The sample collecting device 2a includes a main body 210 having a pair of finger seats 211 formed to be concave to allow a user to easily hold the sample collecting device 2a, and the sample receiving part 220a extending from the main body 210 and having at the top portion spaces 221a and 221b through which a sample (for example, blood) is received by capillary force.

As shown in FIG. 21, the main body 210 of the sample receiving part 220a is, when viewed in the front direction, formed such that the top portion of the main body 210 diverges into three directions. According to a preferred embodiment, the main body 210 may be, when viewed in the front direction, formed to be in a "+" shape.

More specifically, the main body 210 of the sample receiving part 220a is configured to include a first main body 220a-1 having a space 221a for receiving a sample by capillary force, and a second main body 220a-2 formed in the shape resulting from rotating the first main body 220a-1 by 90 degrees with respect to an axis in the front direction of the sample collecting device 2a.

Also, the curvatures of circumference parts 220a-1a and 220a-2a configuring the first and second main bodies 220a-1 and 220a-2 match the curvature of the edge portion 121a of the opening 121. Thereby, when injecting blood onto the measurement layer 110 of the measurement strip through the sample collecting device 2a, a user can securely rest the circumference parts 220a-1a and 220a-2a of the sample receiving part 220a on the edge portion 121a of the opening 121.

The spaces 221a and 221b formed in the top portion of the first and second main bodies 220a-1 and 220a-2 are graved to be in a "V" shape whose width is gradually reduced toward the inside, when viewed in the side direction. Accordingly, the blood can be completely absorbed into the sample receiving part 220a without remaining any blood stains around the circumference parts 220a-1 and 220a-2a.

Also, in the top portion of the sample receiving part 220a, a groove 223 whose diameter is greater than the spacing distance of the spaces 221a and 221b is formed. The groove 223 makes in direct contact with the measurement layer 110 of the measurement strip 1. Accordingly, when blood is injected through the sample collecting device 2a, blood received in the spaces 221a and 221b of the sample receiving part 220 can be rapidly transferred to the measurement layer 110 of the measurement strip 1 through the relatively wide groove 223. According to a preferred embodiment, the groove 223 may be formed in a cone shape whose diameter is gradually reduced from the top portion to the end portion.

FIG. 22 is a view for explaining advantageous effects of the sample collecting device 2a of FIG. 21. In the sample collecting device 2a according to this embodiment, since a groove 223 whose diameter is greater than the spacing distance of the spaces 221a and 221b is formed and the groove 223 makes in direct contact with the upper surface of the measurement layer 110 of the measurement strip 1, blood absorbed in the sample receiving part 220a can be more smoothly and rapidly transferred to the measurement layer 110 of the measurement strip 1. Accordingly, a biodata-measuring apparatus (not shown) can more rapidly acquire measurement results by the measurement strip 1.

Also, in the sample collecting device 2a according to this embodiment, since the curvatures of the circumference parts 220a-1a and 220a-2a match the curvature of the edge portion 121 of the opening 121, it is easy to rest the circumference parts 2201-1a and 220a-2a of the sample receiving part 22a on the edge portion 121a of the opening 121. For example, although a user brings the sample collecting device 2a into contact with the opening 121 of the measurement strip 1 at various angles, he or she can securely rest the sample receiving part on the edge portion 121 of the opening 121 through the circumference parts 220a-1a and 220a-2a of the sample receiving part 220a having a curvature matching that of the edge portion 121a of the opening 121.

Also, in the sample collecting device 2a according to the present invention, since the main body is formed in a "+" shape extending to the up, down, left, and right, a pressing force transferred to the edge portion 121a through the circumference parts 220a-1a and 220a-2a of the sample receiving part 220a can be distributed uniformly in the up, down, left, and right directions. That is, as described above with reference to FIG. 14, when a pressure is applied to a part of the edge portion 121a of the opening 121, the pressed part of the edge portion 121a is compressed downward, and accordingly the F1 flow path directly contributing to serum saturation of the reactive layer 130 of the measurement strip 1 may change. However, in the case of using the sample collecting device 2a according to the present embodiment, a pressing force transferred from the circumference parts 220a-1a and 220a-2a of the sample receiving part 220a configuring the sample collecting device 2a to the edge portion 121a is distributed uniformly in the up, down, left, and right directions, the phenomenon where the edge portion 121 is locally pressed downward can be effectively prevented.

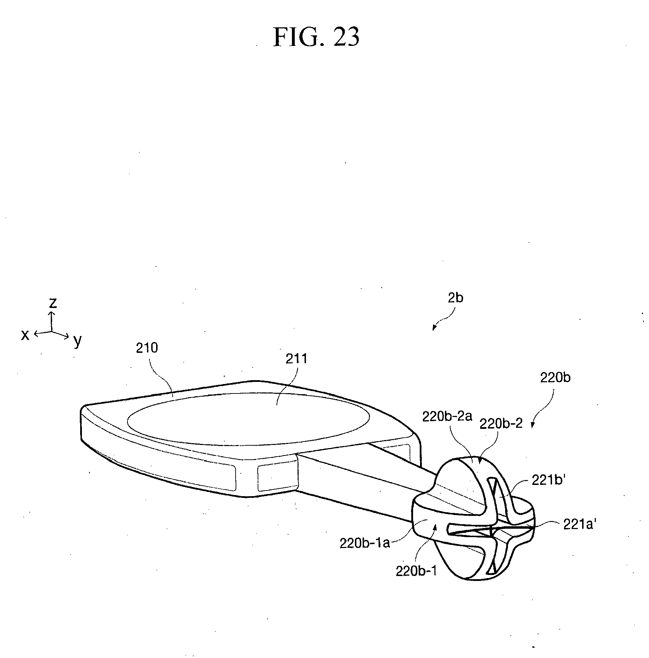

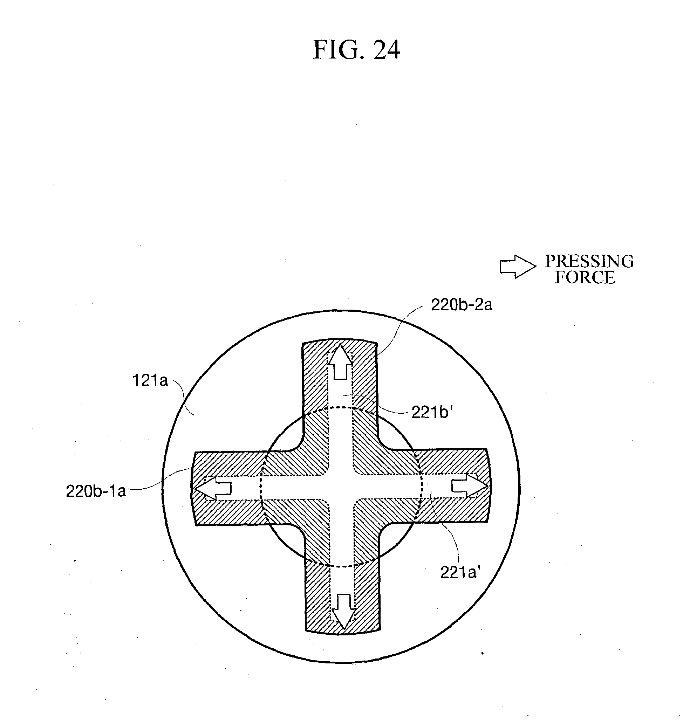

FIG. 23 is a perspective view illustrating a sample collecting device 2b according to another embodiment of the present invention, applied to or included in the biodata-measuring set, and FIG. 24 is a view for explaining advantageous effects of the sample collecting device of FIG. 23.

The sample collecting device 2b according to the present invention has the same structure as the sample collecting device 2 illustrated in FIG. 15, except for the structure of a sample receiving part 220b.

Meanwhile, in FIG. 23, the x-axis direction is defined as the side direction of the sample collecting device 2b, the y-axis direction is defined as the front direction of the sample collecting device 2b, and the z-axis direction is defined as the plane direction of the sample collecting device 2b.

The sample collecting device 2b includes a main body 210 having a pair of finger seats 211 formed to be concave to allow a user to easily hold the sample collecting device 2b, and a sample receiving part 220b having at the top portion spaces 221a' and 221b' for receiving a sample (for example, blood) by capillary force.

As shown in FIG. 23, the sample receiving part 220b is, when viewed in the front direction, formed in such a way to diverge into three directions from the top central portion. According to a preferred embodiment, the sample receiving part 220b may be, when viewed in the front direction, formed to be in a "+" shape.

More specifically, the sample receiving part 220b includes a first main body 220b-1 having a space 221a' for receiving a sample by capillary force, and a second main body 220b-2 formed in the shape resulting from rotating the first main body 220a-1 by 90 degrees with respect to an axis in the front direction of the sample collecting device 2b.

Also, the curvatures of circumference parts 220b-1a and 220b-2a match the curvature of the edge portion 121a of the opening 121. Accordingly, when injecting blood onto the measurement layer 110 of the measurement strip 1 through the sample collecting device 2b, a user can securely rest the circumference parts 220b-1a and 220b-2a of the sample receiving part 220b on the edge portion 121a of the opening 121.

FIG. 24 is a view for explaining advantageous effects of the sample collecting device 2b of FIG. 23. In the sample collecting device 2b according to the present invention, since the curvatures of circumference parts 220b-1a and 220b-2a match the curvature of the edge portion 212a of the opening 121, it is easy to securely rest the circumference parts 220b-1a and 220b-2a of the sample receiving part 220b on the edge portion 121a of the opening 121. For example, although a user brings the sample collecting device 2b into contact with the opening 121 of the measurement strip 1 at various angles, he or she can securely rest the sample collecting part 220b on the edge portion 121a of the opening 121 through the circumference parts 220b-1a and 220b-2a having a curvature matching that of the edge portion 121a of the opening 121.

Also, in the sample collecting device 2b according to the present embodiment, since the sample receiving part is, when viewed in the front direction, formed in a "+" shape extending in the up, down, left and right, a pressing force transferred to the edge portion 121a through the circumference parts 220b-1a and 220b-2a is distributed uniformly in the up, down, left, and right directions. That is, as described above with reference to FIG. 14, when the edge portion 121a of the opening 121 is locally pressed, the pressed part of the edge portion 121a is compressed downward, and accordingly, the F1 flow path directly contributing to serum saturation of the reactive layer 130 of the measurement strip 1 may change. However, in the case of using the sample collecting device 2b according to this embodiment, since a pressing force transferred from the circumference parts 220b-1a and 220b-1b of the sample receiving part 220b to the edge portion 121a is distributed uniformly in the up, down, left, and right directions, the phenomenon where a part of the edge portion 121 is strongly pressed downward can be effectively prevented.

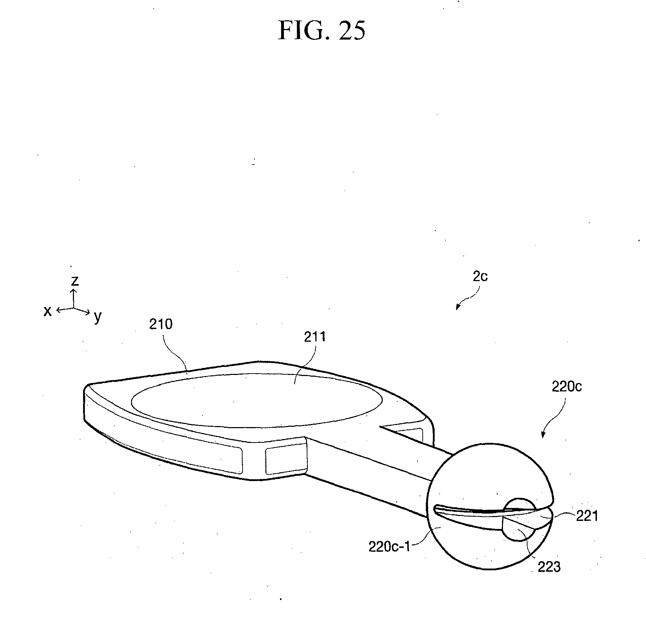

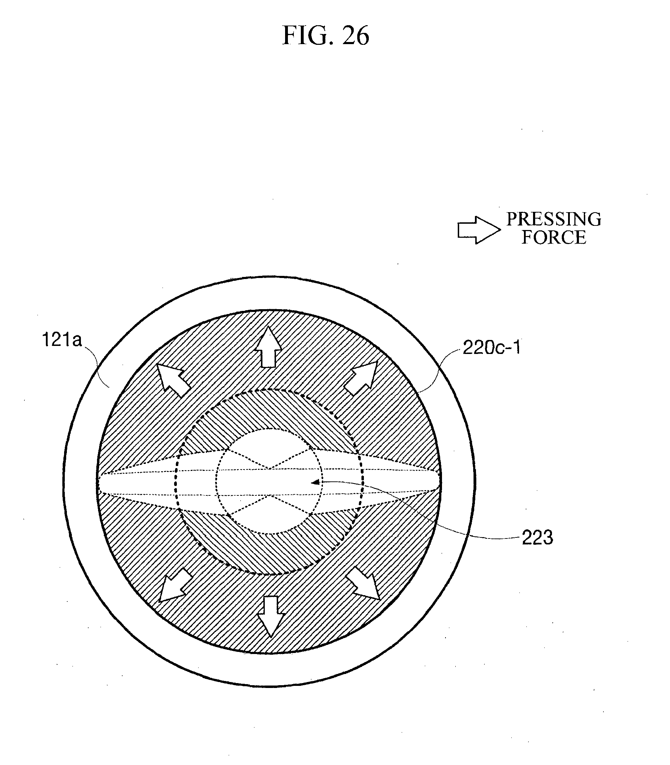

FIG. 25 is a perspective view illustrating a sample collecting device 2c according to another embodiment of the present invention, applied to or included in the biodata-measuring set, and FIG. 26 is a view for explaining advantageous effects of the sample collecting device 2c of FIG. 25.

The sample collecting device 2c according to the present embodiment has the same structure as the sample collecting device 2 illustrated in FIG. 15, except for the structure of a sample receiving part 220c.

Meanwhile, in FIG. 25, the x-axis direction is defined as the side direction of the sample collecting device 2c, the y-axis direction is defined as the front direction of the sample collecting device 2c, and the z-axis direction is defined as the plane direction of the sample collecting device 2c.

The sample collecting device 2c includes a main body 210 having a pair of finger seats 211 formed to be concave to allow a user to easily hold the sample collecting device 2c, and a sample receiving part 220c extending from the main body 210 and having at the top portion a space through which a sample (for example, blood) is received by capillary force.

The body of the sample receiving part 220c is formed in a globe shape.

Also, the curvature of a circumference part 220c-1 configuring the globe shape matches the curvature of the edge portion 121a of the opening 121. Accordingly, when injecting blood onto the measurement layer 110 of the measurement strip 1 through the sample collecting device 2c, a user can securely rest the circumference part 220c-1 of the sample receiving part 220c on the edge portion 121a of the opening 1.

The space 221 formed at the top portion of the sample receiving part 220c is graved to be, when viewed in the side direction, in a "V" shape whose width is gradually reduced toward the inside. Accordingly, when the sample collecting device 2c collects blood, the blood can be completely absorbed into the inside of the sample receiving part 220c without remaining any blood stains around the circumference part 220c-1.

Also, in the top central portion of the sample receiving part 220c, a groove 223 whose diameter is greater than the spacing distance of the space 221 is formed. The groove 223 becomes in direct contact with the measurement layer 110 of the measurement strip 1. Accordingly, when blood is injected through the sample collecting device 2c, the blood can be rapidly transferred to the measurement layer 110 of the measurement strip 1 through the groove 223 having a relatively wide width. According to a preferred embodiment, the groove 223 may be formed in a cone shape whose diameter is gradually reduced from the top portion to the end portion of the sample receiving part 220c.

FIG. 26 is a view for explaining advantageous effects of the sample collecting device 2c of FIG. 25. In the sample collecting device according to the present embodiment, since a groove 223 whose diameter is greater than the spacing distance of the space 221 is formed, blood absorbed in the sample receiving part 220c can be more smoothly and rapidly transferred to the measurement layer 110 of the measurement strip 1. Accordingly, for example, a biodata-measuring apparatus (not shown) can more rapidly acquire measurement results by the measurement strip 1.

Also, in the sample collecting device 2c according to the present embodiment, since the curvatures of the circumference part 220c-1 configuring the globe shape match the curvature of the edge portion 121a configuring the opening 121 of the measurement strip 1, it is easy to rest the circumference part 220c-1 of the sample receiving part 220c on the edge portion 121a of the opening 121.

Also, in the sample collecting device 2c according to the present embodiment, since the sample receiving part 220c is, when viewed in the front direction, formed to be in a globe shape, a downwardly pressing force transferred from the circumference part 220c-1 of the sample collecting device 2c to the edge portion 121a of the opening 121 can be distributed uniformly over the entire area of the edge portion 121. That is, as described above with reference to FIG. 14, when the edge portion 121a of the opening 121 is locally pressed, the pressed part of the edge portion 121 is pressed downward, and accordingly, the F1 flow path directly contributing to serum saturation of the reactive layer 115 of the measurement strip 1 may change. However, in the case of using the sample collecting device 2c according to the present embodiment, since a pressing force transferred from the circumference part 220c-1 of the sample receiving part 220c to the edge portion 121a is distributed uniformly over the entire area of the edge portion 121a, the phenomenon where the edge portion 121a is locally and strongly pressed downward can be effectively prevented.

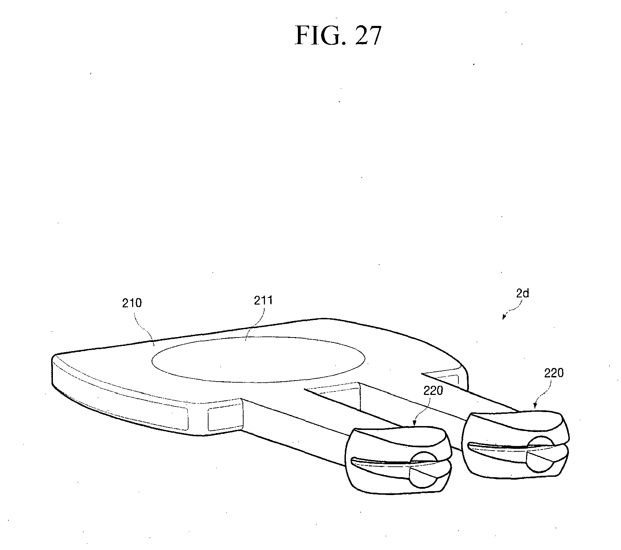

FIG. 27 is a perspective view illustrating a sample collecting device 2d according to another embodiment of the present invention, applied to or included in the biodata-measuring set. The sample collecting device 2d is configured by combining the main body 210 having the concave seats 211 with two or more sample receiving parts each corresponding to one among the embodiments described above. FIG. 27 shows, for convenience of description, a structure where two sample collecting devices 220 each having the structure illustrated in FIG. 15 are connected to a single main body 210, however, the shape of the sample collecting device 2d is not limited to this.

In the case of using the sample collecting device 2d according to the present embodiment, it is possible to simultaneously inject two samples into individual openings 121 of a measurement strip 1.

The above description is provided to assist the reader in gaining a comprehensive understanding of the methods, apparatuses, and/or systems described herein. Accordingly, various changes, modifications, and equivalents of the methods, apparatuses, and/or systems described herein will be suggested to those of ordinary skill in the art. For example, the shapes of the sample receiving part 220 of FIG. 6A, the sample receiving part 220a of FIG. 8A, the sample receiving part 220b of FIG. 9A, and the sample receiving part 220b of FIG. 10A are only exemplary, and various modifications are possible. For example, the sample receiving part may be, when viewed in the front direction, formed in a "Y" shape.

Meanwhile, as described above, a method of measuring biodata is dependent on measuring a change in color or an electrochemical change showing the results of an enzymatic reaction, however, various methods such as using a fluorescent material or radioactivity can be utilized. That is, it is apparent that the method of measuring biodata is not limited to the method of measuring a change in optical color or an electrochemical change.

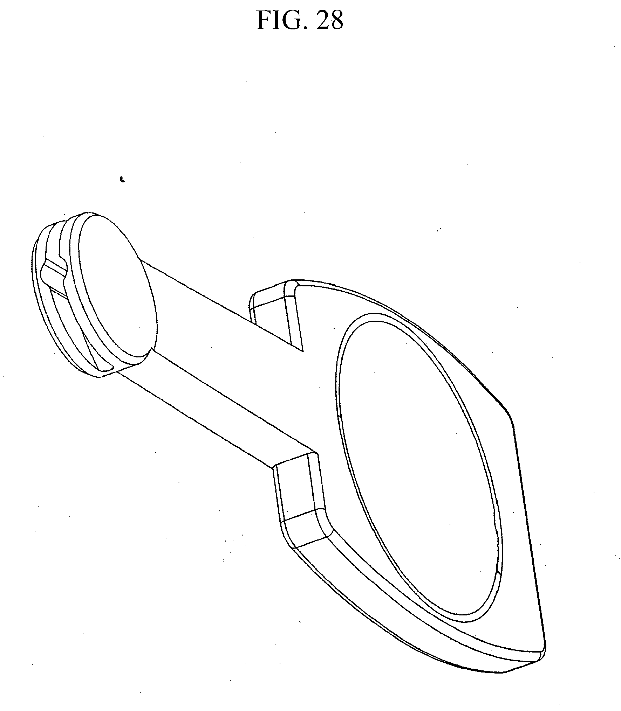

Also, various modified embodiments capable of achieving objects of the present invention are shown in FIGS. 28 through 37. The modified embodiments capable of achieving the objects of the present invention will be schematically described with reference to the associated drawings, as follows.

First, FIG. 28 shows a modified embodiment of the sample collecting device illustrated in the upper part of FIG. 5. Referring to FIG. 28, a groove is graved around the central portion of the space with the "-" shape to ensure a wider area making contact with a measurement layer of a measurement strip, thus increasing injection speed of a sample to thereby accelerate measurement.

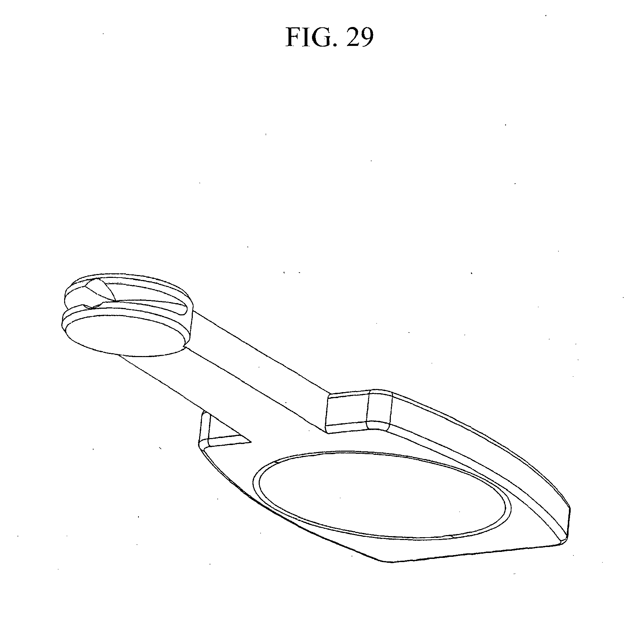

FIG. 29 is a modified embodiment of the sample collecting device of FIG. 28. Referring to FIG. 29, the space of the sample receiving part is formed to be, when viewed in the side direction, in a "V" shape whose width is gradually reduced toward the inside. Accordingly, when blood is collected through the sample collecting device, the blood can be completely absorbed into the inside of the sample receiving part without remaining any blood stains around the circumference part of the opening of a measurement strip. Also, the "V" shape is further advantageous since it can be easily molded upon injection molding.

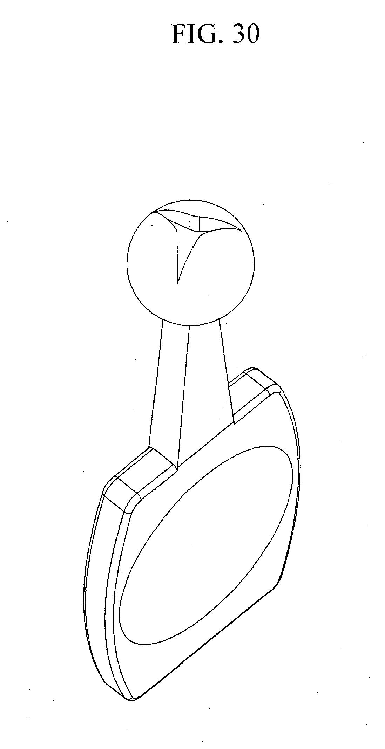

FIG. 30 shows a modified embodiment where a sample receiving part is formed, when viewed in the front direction, in a globe shape such that a downwardly pressing force transferred from the circumference part of the sampling collecting device to the edge portion of the opening is distributed uniformly over the entire area of the edge portion. That is, as described above with reference to FIG. 14, when the edge portion of the opening is locally pressed, the pressed part of the edge portion is compressed downward, and accordingly the F1 flow path directly contributing to serum saturation of the reactive layer 115 of the measurement strip 1 may change. However, in the case of using the sample collecting device according to the present embodiment, since a pressing force transferred from the circumference part of the sample receiving part configuring the sample collecting device to the edge portion is distributed uniformly over the entire area of the edge portion, the phenomenon where a part of the edge part is strongly pressed downward can be completely excluded. Also, since a space is formed in a "Y" shape, the time that a collected sample takes to be injected into the measurement strip can be shortened, compared to the embodiment of FIG. 10A, resulting in acceleration of measurement.

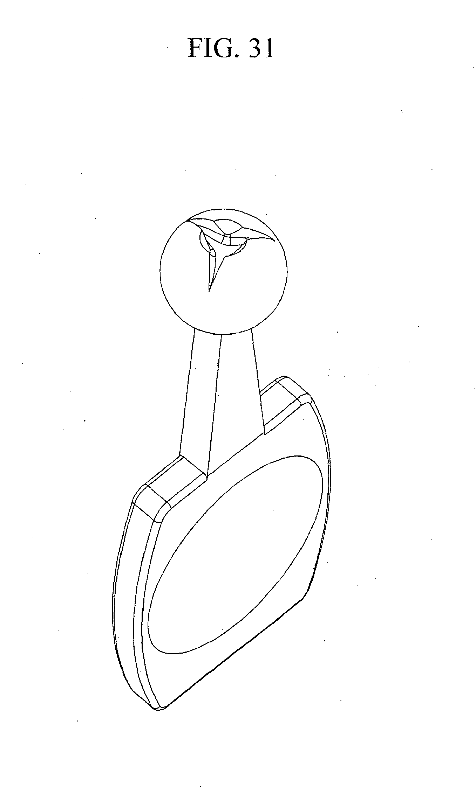

FIG. 31 shows a modified embodiment where a groove is further formed in the central portion of the "Y" shape of FIG. 30 to make a larger contact with an area onto which a sample is applied, which leads to a further acceleration of measurement.

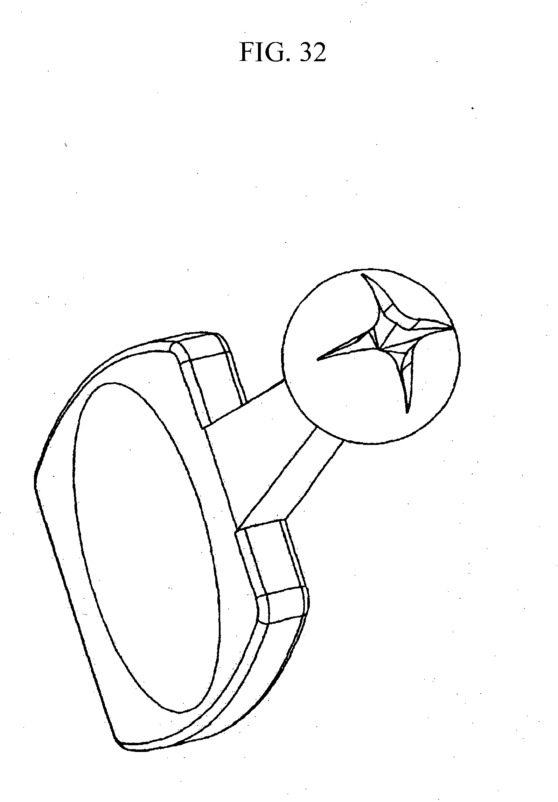

FIG. 32 is different from the configuration illustrated in FIG. 30 in that a space diverging in four directions, not in three directions, is formed, and effects accompanied by the configuration are the nearly same as those of the sample collecting device of FIG. 30.

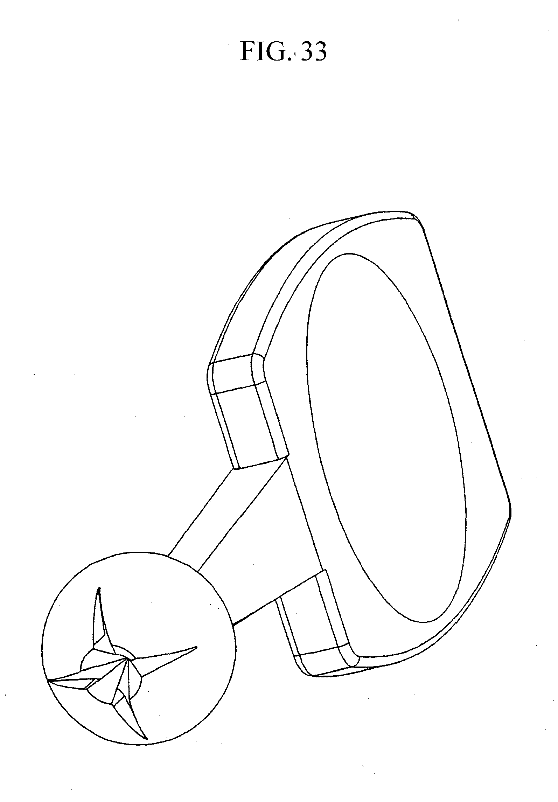

FIG. 33 shows a modified embodiment where a groove is formed in the central portion of the space diverging in four directions, and effects accompanied by the configuration is the nearly same as those of the sample collecting device of FIG. 31.

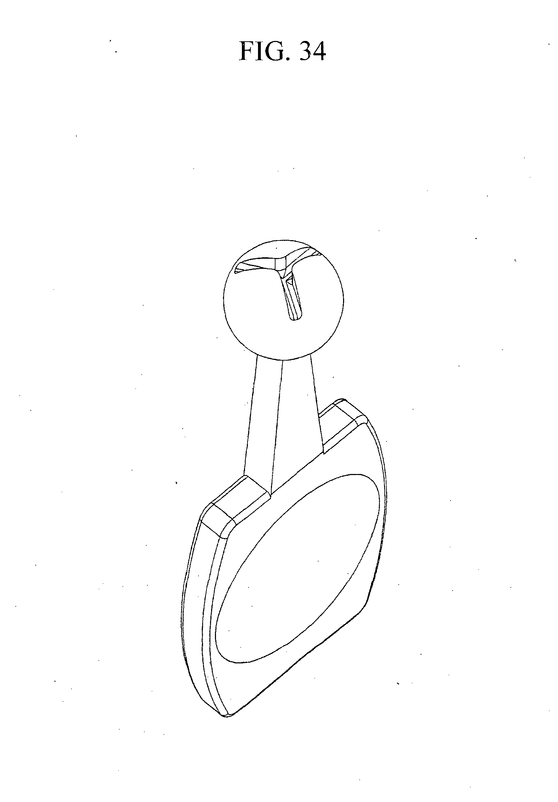

FIG. 34 is different from the configuration illustrated in FIG. 30 in that a space into which a sample is received is formed with a constant width, instead of having a "V"-shaped width that is reduced toward the inside of the sample receiving part, and effects accompanied by the configuration is the nearly same as those of the sample collecting device of FIG. 30.

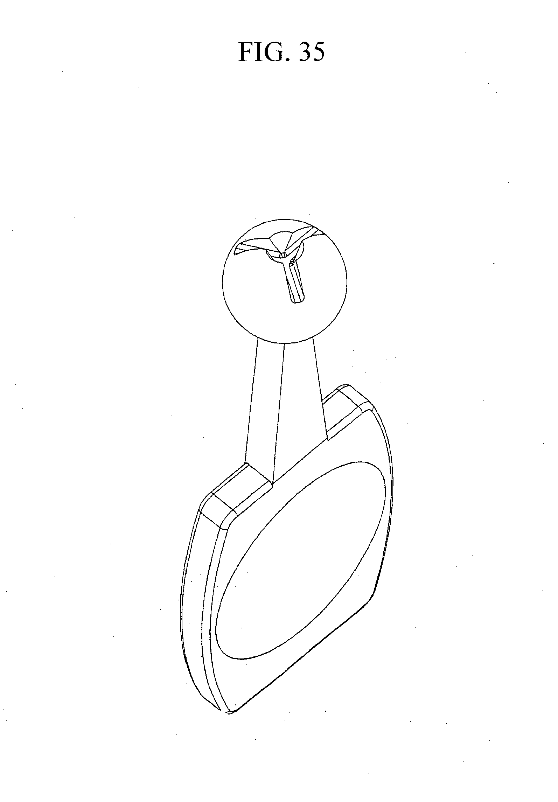

FIG. 35 is different from the configuration illustrated in FIG. 31 in that a space into which a sample is received is formed with a constant width, instead of having a "V"-shaped width that is reduced toward the inside of the sample receiving part, and effects accompanied by the configuration is the nearly same as those of the sample collecting device of FIG. 31.

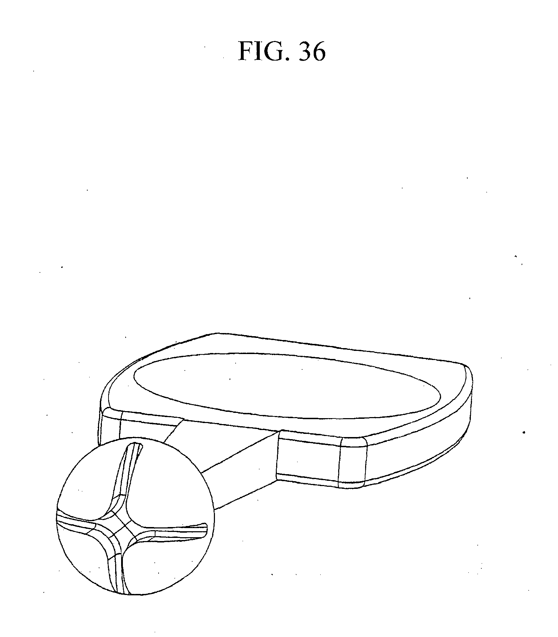

FIG. 36 is different from the configuration illustrated in FIG. 32 in that a space into which a sample is received is formed with a constant width, instead of having a "V"-shaped width that is reduced toward the inside of the sample receiving part, and effects accompanied by the configuration is the nearly same as those of the sample collecting device of FIG. 32.

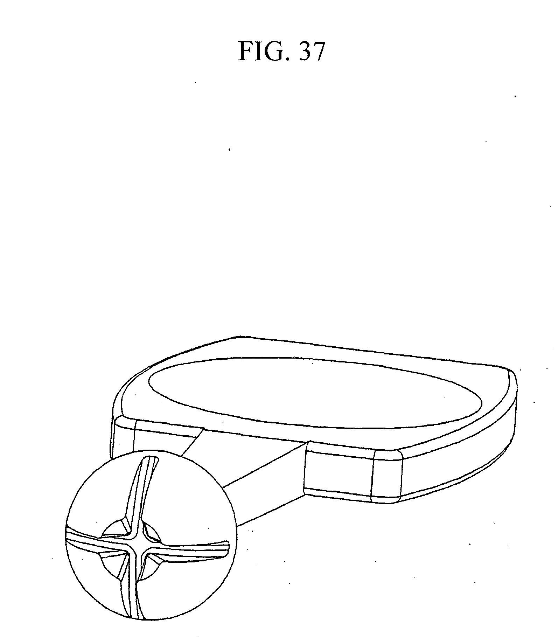

FIG. 37 is different from the configuration illustrated in FIG. 33 in that a space into which a sample is received is formed with a constant width, instead of having a "V"-shaped width that is reduced toward the inside of the sample receiving part, and effects accompanied by the configuration is the nearly same as those of the sample collecting device of FIG. 33.

Also, each of the sample collecting devices illustrated in FIGS. 12 through 21 may be provided together with a measurement strip that is used to measure biodata, as a biodata-measuring set, which is not illustrated in the drawings.

Also, a process and principle in which each of the sample collecting devices illustrated in FIGS. 28 through 37 injects a sample onto a measurement strip will be understood from the above descriptions with reference to FIGS. 15 through 27, which are not illustrated in the drawings.

Also, in FIG. 28, the groove of the sample receiving part is formed with a constant width, however, the groove of the sample receiving part may be formed to have a "V"-shaped width that is reduced toward the inside of the sample receiving part, as illustrated in FIG. 29. Likewise, in FIG. 29, the groove of the sample receiving part is formed to have a "V"-shaped width that is reduced toward the inside of the sample receiving part, however, the groove of the sample receiving part may be formed with a constant width. This can be applied in the same way to the embodiments illustrated in FIGS. 31, 33, 35, and 37.

Meanwhile, in the embodiments described above, the sample receiving part is in a globe shape, or the top central portion of the sample receiving part is, when viewed in the front direction, formed to diverge in at least two directions. However, the present invention is not limited to these.

Also, the shape of the space is formed to be in various shapes, as well as a "-" shape or a diverging shape. For example, the shape of the space may be formed in a curved shape, such as a "~" shape.

A number of examples have been described above. Nevertheless, it will be understood that various modifications may be made. For example, suitable results may be achieved if the described techniques are performed in a different order and/or if components in a described system, architecture, device, or circuit are combined in a different manner and/or replaced or supplemented by other components or their equivalents. Accordingly, other implementations are within the scope of the following claims.

## Claims

1. A device for collecting a sample and injecting the sample onto a measurement strip, comprising:
a main body (210); and
a sample receiving part (220) protruding from a first end of the main body (210) and having in a top portion a space (221) for receiving the sample by capillary force, wherein an inner wall of the space is treated with a surfactant, wherein the space is formed to diverge in at least two directions from a top central portion of the sample receiving part (220), when viewed in a front direction,
wherein the sample receiving part (220) is formed to diverge in at least two directions from a top central portion of the sample receiving part, when viewed in a front direction, and
**characterised in that** the sample receiving part (220) includes an upper portion formed concavely toward the inside of the sample receiving part, a lower portion formed concavely toward the inside of the sample receiving part while facing the upper portion, and a circumference part connecting the upper portion to the lower portion and formed in a curved shape.

2. A device as set forth in claim 1, wherein the sample receiving part (220) is a pair of receiving parts which is open on right, left and bottom sides,
and wherein a curvature of the sample receiving part (220) is configured such that an end of the sample receiving part matches a hole formed in a reaction zone of a measurement strip and is in slight contact with the reaction zone.

3. The device as set forth in claim 1, wherein in the top central portion of the sample receiving part, a groove (223) is formed having a diameter greater than the spacing distance of the space (221).

4. The device as set forth in claim 1, wherein the top portion of the sample receiving part protrudes in an arc shape or in a globe shape.

5. The device as set forth in claim 1, wherein the space is formed in a "-" or "+" when viewed in a front direction.

6. The device as set forth in one of claims 1 to 5, wherein in the top central portion of the sample receiving part, a groove (223) whose diameter is greater than a spacing distance of the space is formed.

7. The device as set forth in one of claims 1 to 5, wherein the space (221) is graved in a shape whose width is gradually reduced toward the inside of the sample receiving part (220) when viewed in a side direction.

8. The device as set forth in claim 6, wherein the space (221) is graved in a shape whose width is gradually reduced toward the inside of the body of the sample receiving part (220), when viewed in a side direction.

9. The device as set forth in claim 6, wherein the groove (223) is formed in a shape whose diameter is gradually reduced toward the inside of the sample receiving device.

10. The device as set forth in one of claims 1 to 5, wherein a volume of the space (221) is determined by a volume of the received sample.

11. The device as set forth in claims 1 to 5, wherein the sample receiving part (220) is made of a transparent material.

12. A biodata-measuring set comprising:
a measurement strip (1) including a sample receiving area onto which a sample is injected; and
the sample collecting device of one of claims 1 to 8.

## Patentansprüche

1. Vorrichtung zum Nehmen einer Probe und Injizieren der Probe auf einen Messstreifen, die Folgendes umfasst:
einen Hauptkörper (210); und
einen Probenaufnahmeteil (220), der von einem ersten Ende des Hauptkörpers (210) vorsteht und in einem oberen Teil einen Raum (221) zur Aufnahme der Probe durch Kapillarkraft aufweist, wobei eine Innenwand des Raums mit einem Tensid behandelt ist, wobei der Raum so gestaltet ist, dass er in einer Frontrichtung betrachtet in wenigstens zwei Richtungen von einem oberen mittleren Teil des Probenaufnahmeteils (220) divergiert,
wobei der Probenaufnahmeteil (220) so ausgebildet ist, dass er in einer Frontrichtung betrachtet in wenigstens zwei Richtungen von einem oberen mittleren Teil des Probenaufnahmeteils divergiert, und
**dadurch gekennzeichnet, dass** der Probenaufnahmeteil (220) einen konkav in Richtung auf die Innenseite des Probenaufnahmeteils ausgebildeten oberen Teil, einen konkav in Richtung auf die Innenseite des Probenaufnahmeteils ausgebildeten, dem oberen Teil zugewandten unteren Teil und einen den oberen Teil mit dem unteren Teil verbindenden und gekrümmt ausgebildeten Umfangsteil aufweist.

2. Vorrichtung nach Anspruch 1, wobei der Probenaufnahmeteil (220) ein Paar Aufnahmeteile ist, das auf der rechten, linken und unteren Seite offen ist,
und wobei eine Krümmung des Probenaufnahmeteils (220) so konfiguriert ist, dass ein Ende des Probenaufnahmeteils mit einem in einer Reaktionszone eines Messstreifens ausgebildeten Loch übereinstimmt und mit der Reaktionszone in geringem Kontakt ist.

3. Vorrichtung nach Anspruch 1, wobei im oberen mittleren Teil des Probenaufnahmeteils eine Nut (223) mit einem Durchmesser ausgebildet ist, der größer ist als das Abstandsmaß des Raums (221).

4. Vorrichtung nach Anspruch 1, wobei der obere Teil des Probenaufnahmeteils bogenförmig oder kugelförmig vorsteht.

5. Vorrichtung nach Anspruch 1, wobei der Raum in einer Frontrichtung gesehen die Form eines "-" oder "+" hat.

6. Vorrichtung nach einem der Ansprüche 1 bis 5, wobei im oberen mittleren Teil des Probenaufnahmeteils eine Nut (223) ausgebildet ist, deren Durchmesser größer ist als ein Abstandsmaß des Raums.

7. Vorrichtung nach einem der Ansprüche 1 bis 5, wobei der Raum (221) in einer Form graviert ist, deren Breite in einer Seitenrichtung gesehen in Richtung auf die Innenseite des Probenaufnahmeteils (220) allmählich abnimmt.

8. Vorrichtung nach Anspruch 6, wobei der Raum (221) in einer Form graviert ist, deren Breite in einer Seitenrichtung gesehen in Richtung auf die Innenseite des Körpers des Probenaufnahmeteils (220) allmählich abnimmt.

9. Vorrichtung nach Anspruch 6, wobei die Nut (223) in einer Form ausgebildet ist, deren Durchmesser in Richtung auf die Innenseite der Probenaufnahmevorrichtung allmählich abnimmt.

10. Vorrichtung nach einem der Ansprüche 1 bis 5, wobei ein Volumen des Raums (221) durch ein Volumen der aufgenommenen Probe bestimmt wird.

11. Vorrichtung nach einem der Ansprüche 1 bis 5, wobei der Probenaufnahmeteil (220) aus einem transparenten Material gefertigt ist.

12. Biodatenmesssatz, der Folgendes umfasst:
einen Messstreifen (1) mit einem Probenaufnahmebereich, auf den eine Probe injiziert wird; und
die Probenahmevorrichtung nach einem der Ansprüche 1 bis 8.

## Revendications

1. Dispositif pour prélever un échantillon et l'injecter sur une bande de mesure, comprenant :
un corps principal (210) ; et
une partie recevant l'échantillon (220) dépassant d'une première extrémité du corps principal (210) et ayant, dans une partie supérieure, un espace (221) pour recevoir l'échantillon par capillarité, une paroi intérieure de l'espace étant traitée avec un agent tensio-actif, l'espace étant formé pour diverger dans au moins deux directions par rapport à une partie centrale supérieure de la partie recevant l'échantillon (220), lorsqu'il est observé dans une direction avant ;
dans lequel la partie recevant l'échantillon (220) est formée pour diverger dans au moins deux directions par rapport à une partie centrale supérieure de la partie recevant l'échantillon, lorsqu'elle est observée dans une direction avant, et
**caractérisé en ce que** la partie recevant l'échantillon (220) comprend une partie supérieure ayant une forme concave vers l'intérieur de la partie recevant l'échantillon, une partie inférieure ayant une forme concave vers l'intérieur de la partie recevant l'échantillon tout en faisant face à la partie supérieure, et une partie circonférentielle reliant la partie supérieure à la partie inférieure et ayant une forme incurvée.

2. Dispositif selon la revendication 1, dans lequel la partie recevant l'échantillon (220) est une paire de parties de réception qui est ouverte sur les côtés droit, gauche et inférieur,
et dans lequel une courbure de la partie recevant l'échantillon (220) est configurée de telle sorte qu'une extrémité de la partie recevant l'échantillon correspond à un trou formé dans une zone de réaction d'une bande de mesure et est en léger contact avec la zone de réaction.

3. Dispositif selon la revendication 1, dans lequel, dans la partie centrale supérieure de la partie recevant l'échantillon, est formée une rainure (223) ayant un diamètre plus grand que la distance d'espacement de l'espace (221).

4. Dispositif selon la revendication 1, dans lequel la partie supérieure de la partie recevant l'échantillon dépasse en une forme d'arc ou en une forme de globe.

5. Dispositif selon la revendication 1, dans lequel l'espace est en forme de "-" ou de "+" lorsqu'il est observé dans une direction avant.

6. Dispositif selon l'une des revendications 1 à 5, dans lequel, dans la partie centrale supérieure de la partie recevant l'échantillon, est formée une rainure (223) dont le diamètre est plus grand qu'une distance d'espacement de l'espace.

7. Dispositif selon l'une des revendications 1 à 5, dans lequel l'espace (221) est gravé en une forme dont la largeur diminue progressivement vers l'intérieur de la partie recevant l'échantillon (220) lorsqu'il est observé dans une direction latérale.

8. Dispositif selon la revendication 6, dans lequel l'espace (221) est gravé en une forme dont la largeur diminue progressivement vers l'intérieur du corps de la partie recevant l'échantillon (220), lorsqu'il est observé dans une direction latérale.

9. Dispositif selon la revendication 6, dans lequel la rainure (223) a une forme dont le diamètre diminue progressivement vers l'intérieur du dispositif recevant l'échantillon.

10. Dispositif selon l'une quelconque des revendications 1 à 5, dans lequel un volume de l'espace (221) est déterminé par un volume de l'échantillon reçu.

11. Dispositif selon les revendications 1 à 5, dans lequel la partie recevant l'échantillon (220) est constituée d'un matériau transparent.

12. Ensemble de mesure de données biographiques comprenant .
une bande de mesure (1) comportant une zone de réception d'échantillon sur laquelle est injecté un échantillon ; et
le dispositif de prélèvement d'échantillon selon l'une des revendications 1 à 8.
